# EUROPEAN PATENT SPECIFICATION

(11) **EP 3 788 372 B1**
(45) Date of publication and mention of the grant of the patent: **26.10.2022**
(21) Application number: 19719530.8
(22) Date of filing: 29.04.2019
(51) Int. Cl.: G01N 33/543, G01N 33/58

(54) **IMMUNOASSAY FOR AN AUTOMATED SYSTEM**
IMMUNOASSAY FÜR EIN AUTOMATISIERTES SYSTEM
DOSAGE IMMUNOLOGIQUE POUR UN SYSTÈME AUTOMATISÉ

(30) Priority: 02.05.2018 EP 18170409
(43) Date of publication of application: 10.03.2021
(73) Proprietor: Hahn-Schickard-Gesellschaft für angewandte Forschung e.V., 78052 Villingen-Schwenningen (DE)
(72) Inventor: JOHANNSEN, Benita-Andrea, 79114 Freiburg (DE); KLEIN, Vanessa, 79110 Freiburg (DE); MITSAKAKIS, Konstantinos, 79106 Freiburg (DE)
(74) Representative: Hertin und Partner Rechts- und Patentanwälte PartG mbB
(86) International application number: PCT/EP2019/060864
(87) International publication number: WO 2019/211218

(56) References cited:
- EP-A2- 0 490 358
- WO-A1-2008/056165
- GB-A- 2 045 431
- US-A- 5 447 870
- US-A1- 2002 004 199
- O. STROHMEIER ET AL: "Centrifugal microfluidic platforms: advanced unit operations and applications", CHEMICAL SOCIETY REVIEWS, vol. 44, no. 17, 1 January 2015 (2015-01-01), pages 6187-6229, XP055423852, ISSN: 0306-0012, DOI: 10.1039/C4CS00371C
- FERNANDEZ-BALDO M A ET AL: "Determination of ochratoxin A in apples contaminated with Aspergillus ochraceus by using a microfluidic competitive immunosensor with magnetic nanoparticles.", ANALYST, vol. 136, no. 13, 7 July 2011 (2011-07-07) , pages 2756-2762, XP002782579, DOI: 10.1039/c1an15148g
- Y. ZHAO ET AL: "C-reactive protein and interleukin 6 microfluidic immunoassays with on-chip pre-stored reagents and centrifugo-pneumatic liquid control", LAB ON A CHIP, vol. 17, no. 9, 1 January 2017 (2017-01-01), pages 1666-1677, XP055488356, ISSN: 1473-0197, DOI: 10.1039/C7LC00251C

## Description

The invention relates to a method for detecting a target molecule in a sample, comprising the sequential steps of (a.) incubating at the same time in a buffer solution in a reaction well (i.) the sample suspected to contain one or more target molecules, (ii.) solid phase-coupled capture molecules suitable for binding to the target molecules, wherein the capture molecules are coupled to beads (capture-beads), (iii.) a defined amount of detection molecules suitable for binding to the target molecules and/or the capture molecules, wherein the detection molecules are coupled to beads (detection-beads), wherein the capture-beads are separable from the detection-beads, (b.) separating in the reaction well the capture-beads from the buffer solution comprising unbound detection-beads, and (c.) detecting the remaining unbound detection-beads in the buffer solution, which has been separated from the capture-beads, wherein the detection of remaining unbound detection-beads occurs in the same reaction well as the incubation and separation, wherein the method does not comprise a washing step.

### BACKGROUND OF THE INVENTION

There is an increasing need for rapid, specific and sensitive diagnostic tests on the site where patient management is done, especially in cases of emergencies, or time-critical decision making. For example, one of the big healthcare challenges comes with the rising of antimicrobial resistant (AMR) bacteria, where a major cause is assumed to be the overuse of antibiotics for acute febrile illnesses [1]. One big step towards decreasing the issue of AMR is the use of a rapid diagnostic test that is also able to distinguish between bacterial and viral infections [1].

Nucleic acid amplification testing (NAAT) can be used for this, however it is a procedure that includes tedious sample preparation in its manual configuration. Furthermore, only a very limited number of platforms offer truly multi-pathogen panel(s). In addition, the NAAT aims to specifically diagnose the pathogen, whereas for the first prescription decision if antibiotics are needed, it is only important to differentiate between viral or bacterial infection.

On the other hand, protein markers such as C-reactive protein (CRP) and procalcitonin (PCT) have been often used as indicators of sepsis, and/or inflammation in general (CRP) [2], [3], [4]. Especially in the case of sepsis it is of vital importance that the result is derived as rapidly as possible, to avoid severe complications and improper antibiotic prescription. Recent studies indicate that the inclusion of more than one protein markers and co-assessment of their levels in the diagnostic procedure may have major impact on the differential diagnosis between bacterial and viral infections, and consequently, the decrease of unnecessary prescription of antibiotics [5]. In cancer diagnosis, the use of protein biomarkers is gradually increasing, either at the laboratory or at the point of need, where biomarker screening enables a more personalized patient screening and treatment [6]. Some indicative examples of biomarkers are: Prostate Specific Antigen (PSA) in prostate cancer [7], and CA 15-3, CA 125 and sHER2 in breast cancer [8].

In cardiovascular diseases, again, symptoms such as pain on the chest, or at the back are often easy to misunderstand or misinterpret, thereby missing the opportunity to visit a clinic. The complexity of the cardiovascular diseases requires the detection of multi-panel markers [9].

Furthermore in the field of oral infections, the monitoring of the bacterial flora of the oral cavity is typically done via bacteria identification (NAAT). However, it has been shown that the detection and (semi)quantitative characterization of host protein biomarkers is an important factor to be co-assessed with the bacterial flora, thereby contributing to the long-term personalized monitoring of the patient. Several biomarkers have been identified for periodontitis, which should be monitored as a multi-marker panel for a reliable personalized monitoring [10].

The implementation of the aforementioned protein marker detection through immunoassays can be typically done in a central laboratory. However, for reasons that have to do with reduction of costs and avoidance of sample transport from the point of collection to the point of analysis, it is highly desired that these tests are integrated on platforms and performed in a fully automated way, preferably in a way that the user only adds the sample. Fully automated tests also enable the implementation outside a central laboratory in Point-of-Care (PoC) settings where the user experience level may range from well- to limited-trained. These fully automated PoC tests also include state-of-the-art biomarkers for the intended application field, thereby increasing the quality of patient diagnostics.

Immunoassays for an automated system should have the following properties, which are key elements to create automated systems that are also able to be used at the point of care:
- Minimum workflow steps (to subsequently allow minimum complexity on the automated system)
- Minimum number of reagents
- Short incubation time
- Potential for quantitative results
- Potential for multiplexing, even when the different markers are present in broadly different clinical concentration ranges
- Flexibility and easy adaptability to detect markers requiring different assay specifications

The first immunoassay was described in 1959 [11]. Since then, a wide variety of publications and patents can be found on the topic of immunoassay processes and applications.

S. K. Vashist et al. [12] describe a rapid sandwich one-step enzyme-linked immunosorbent assay (ELISA) for the detection of CRP. The assay only requires an incubation step, two washing steps, adding of the tetramethylbenzidine (TMB)-substrate to generate the to be detected colorimetric signal. Although the assay described in this publication is introduced as rapid and with a high potential for the development of cost-effective *in vitro* diagnostic kits [12] it still needs two washing steps and therefore also requires the use of corresponding washing buffers. Furthermore, the optimum of the performance of the assay during incubation is stated to be at 30 min, which is too long for a rapid PoC test.

However, such an immunoassay method is difficult to implement on a fully automated system, such as a centrifugal microfluidic platform, as is evident from Y. Zhao et al. [13]. To automate the immunoassay for the biomarkers CRP and interleukin-6 (IL-6) on a centrifugal microfluidic platform, a variety of unit operations like three pneumatic pumping structures with an included aliquoting are needed. The assay that was automated in [13] is a bead-based immunoassay that uses a sandwich concept with an absorption based detection method. For a fully automated system, all of the reagents that will be needed during the assay like washing buffer, antibodies, substrate buffer or stop buffer have to be pre-stored on the cartridge. This is directly linked to the working steps that are included in the immunoassay working process. The more steps an assay has, the more unit operations are needed, which leads to an increased footprint and complexity of the microfluidic structures. However, the method presented in [13] is not suitable to reduce the footprint and high number of unit operations, which makes it a complex process for a full automation. The large footprint restricts the degree of throughput (number of samples per test run) and multiplexing (number of detectable biomarkers) on one cartridge. The complexity to automate was due to the fact that several assay steps, including also several different assay reagents, were needed to be done sequentially and washing steps were absolutely required. To facilitate automated assays, it would be a great advantage to develop a single-step assay, that does not necessarily require a wahing step after separation, but instead straightforward detection of the bound-free phase, all in the same chamber. In such a context, the automation in a (centrifugal) microfluidic platform would become increasingly easier, more robust and cost-efficient. Furthermore, when it comes to immunoassay automation in microfluidic systems, the reduction of assay steps corresponds to a reduction of chambers and channels leading to a lower risk for loss of proteins due to adsorption or other reasons.

US4,868,131A [14] describes an immunoassay process where in one step an analyte antigen reacts with antibodies immobilized on water-insoluble microparticles. After the competitive antigen-antibody reaction is completed, the reaction solution is spotted on an integral multilayered analysis element with a porous layer on top, a detection layer and a water-impermeable and transparent bottom layer. The microparticles are too big to pass the first porous layer and only the labeled surplus bound-free antigens can pass to the detection layer and are stopped there. After the filtration step is finished the detection layer is read out. This assay was used for a colorimetric detection of anti-immunoglobulin G (anti-IgG) in serum. The assay process introduced in US4,868,131A reports the detection of the bound-free phase (liquid phase/supernatant), however it is reported in combination with a necessary dry integral multilayered membrane to separate the solid phase from the bound-free phase and to detect the bound-free phase. This restricts the application possibilities. The membrane sorts the reagents according to its pore size only by capillary and diffusion forces. This could be prone to be unspecific or inaccurate. The two incubation steps, one in tube and one on the membrane surface, introduce new challenges for assay automatization in standard environments like microtiter plates or also automated platforms like the above described centrifugal microfluidic platform, or any other non-membrane based configurations.

US4,659,678A [15] describes an immunoassay format with at least one part being a monoclonal or fragmented antibody where a complex is formed between the antigen in the sample and two or more antibodies. The complex is bound non-covalently to a solid phase that can be planar surfaces, like wall-coatings, or particles. This patent claims an assay where monoclonal antibodies are used to reduce the unspecific binding on the solid surface and with that reduce the signal noise. Furthermore, only non-covalent bonding is used to bind the complex to the solid support. The complex on the support is detected. The support can be beads and if desired magnetic particles so that the phase separation can be conducted by magnetic forces. However, the assay of US4,659,678A requires at least one monoclonal antibody, and such antibodies are not for all applications the best choice. Furthermore, this assay can only be used as a sandwich type. This restricts the application portfolio, because a competitive assay might be required for some specific applications. The example assays presented in this patent have long incubation times of 2 h to more than 4 h and need a washing step.

Furthermore, in US4,244,940A [16] an one-step sandwich assay is described in which the sample contains the to be detected ligand, a labeled receptor and an unlabeled receptor that is covalently bound to a solid phase. All these components are reacting in one step in an aqueous solution to form the complex. After the reaction, the solid and liquid phases are separated and the signal is detected in either phase. However, this assay has a long incubation time of 2 h. This patent also describes assays that need filter paper or plastic backed absorbent paper, which restricts the applications that this assay can be used for. It also needs a high number of reagents, which makes this assay difficult to automate.

US5,840,501 [17] describes an immunoassay for the detection of the complex forms of prostate-specific antigen (PSA) in blood and simultaneously removing uncomplexed PSA. The method is decribed as a conventional immunoassay known by skilled users, wherein a separation by antibody-conjugation is used. However, an additional third antibody is needed to remove the uncomplexed PSA. The described assay requires a washing step. Washing in general is a procedure that is prone to errors, requires additional reagents and increases the duration of the assay due to the additionally performed steps. The assay of US5,840,501 is described in a way that the amount of the label in either the bound or unbound phase can be measured and that the label can be measured in each of the separated fractions. However, due to the presence of a washing step, the method as described in US5,840,501 is by definition not applicable in the unbound phase because the washing step would remove the unbound phase itself. Also, US5,840,501 appears to exclusively refer to/cite assays that contain washing steps and, consequently, are also by definition not compatible with the unbound phase detection as proposed by the method of the present invention, because with a washing step the unbound phase is lost. Therefore, there seems to be a contradiction between the assumption-statement of the authors of US5,840,501 and the documented experimental procedures. This shows that it is not obvious to apply the detection in the unbound phase. In addition to the necessity of a washing step, US5,840,501 shows long incubation time of this assay (the incubation time alone, even in the automated system, was 38 minutes and 78 minutes) as well as sequential assay steps. Thus, although the US5,840,501 reports applicability in automated system, it is barely implementable in point-of-care applications.

US5,447,870 [18] describes an immunochromatographic assay that uses a chromatographic solid support with a flocculating agent immobilized thereon and bioaffinity separation to capture an analyte from a complex sample matrix. This method is by definition different from methods where all involved components react in liquid.

The method of US 5,447,870 refers to measuring a free label in a reaction mixture or bound on a support. US5,447,870 describes an immunochromatographic assay that uses a chromatographic solid support with a flocculating agent immobilized thereon and bioaffinity separation to capture an analyte from a complex sample matrix. The removal/separation of the solid from the liquid environment and subsequent detection of the label is a common practice. However, there is a need in the art for an assay that requires no additional material for the separation and no washing after the separation and before the detection, while these two processes take place in the same liquid environment. In other words, it would be a great advantage over the state of the art to develop an assay that enables detection of the labeled molecules of the bound-free phase in liquid environment after separation form the the solid phase without a washing step, while the solid and the liquid phase are present in the same well or container.

Furthermore, US5,447,870 reports that the free label is eluted through the column, and that contaminants are washed away and the analyte is detected by using any number of well-known assay protocols. This clearly indicates that after the capturing, the describe method requires a washing step to be performed prior to detection.

Also, US5,447,870 focuses on the separation method only, and includes, *ex situ* assay/incubation (prior to column injection) and *ex situ* collection of components-to-be-detected (after washing/elution). However, it would be advantageous to develop a method that involves an incubation, *in situ* separation of solid and liquid phase without an intermediate wash and *in situ* detection, all being done in the same reaction well.

Additionally to these facts is the extra working step of transferring the liquid onto a membrane, which presents an additional challenge to automize a robust and reproducible transfer from liquid assay to a membrane. Such examples of US 5,447,870 need several incubations, prior to injection on the membrane, that sum up to 50 minutes. The automation of such assays that require more than one incubation time with different reagents is more complex and disadvantageous.

O. Strohmeier et. al (Chem. Soc. Rev. 2015, vol.44, no. 17, pages 6187-6229) [19] summarize solutions for the automation of immunoassays on centrifugal microfluidic platforms, which all require relatively long time frames to provide a result. It would be advantageous to develop an assay with a lower complexity to allow a higher degree of automation and faster generation of results in comparison with the assays described therein. A desired assay would reduce the number of reagents needed for the automation to enable a reduced footprint on centrifugal systems and less challenges for the storage of the reagents on the disposable. Furthermore, it would be advantageous to simplify the protocol complexity. Such advantages are achieved by the method of the invention described herein, since the assay/incubation, separation, and wash-free detection of the bound-free phase can be achieved in a single reaction well. Furthermore, the advantages of the method of the present invention allow a minimum number of microfluidic structures necessary for automation, thereby reducing the risk of protein loss due to adsorption on microchannels and chambers, while retaining the properties of heterogeneous immunoassays. An important advantage of the method described herein is the reduction of the dependency on the separation process, which is always needed in heterogeneous systems. The in this invention described assay does not rely on the total amount of detection label, but measures the concentration in the liquid phase. This simplifies the automation and makes the system more robust.

WO 2008/056165 A1 [20] describes a saturation assay, which can be an agglutination assay that separates bound and unbound fraction with a porous medium e.g. membrane comparable to lateral flow assay.

The lateral flow assay format is by structure and function different from the method of the present invention, which refers to liquid based ELISA assays. The method of WO 2008/056165 A1 is based on measurement of the free or unbound labelled reagent fraction, but the method requires concentration of the unbound labeled material to not lose sensitivity, since lateral flow assays by nature have the disadvantage to be not sufficiently sensitive. Thus, the undbound fraction needs to be additionaly immobilized in a designated trapping zone which means that, structurally, the labelled reagent fraction is not detected in the unbound liquid phase but is detected only after an additional immobilization step is performed and the detection takes place on a solid support. This additional step required to be able to achieve the needed sensitivity leads to the obvious disadvantage of non-specific surface binding in this trapping zone. In contrast, the method described herein is structurally and functionally clearly different and advantageous in that detection occurs in the unbound liquid phase, in the same liquid fraction where the assay/binding reaction took place and where the solid phase was separated. Thereby, the risk of non-specific interactions of the "useful" unbound labeled molecules with any solid support surface is minimized, as no concentration/condensation measures are required to achieve the necessary sensitivity.

Additionally, the fact that the separation in the method of WO 2008/056165 A1 is taking place using a membrane, is disadvantageous because membranes are an additional factor that can cause unspecific binding of the protein on the membrane material and proteins can be lost in the membrane due to the variation of pore size typically found in membrane material. The assay format of WO 2008/056165 A1 is also restricted to testing of molecules with larger sizes, because too small molecules are not captured in the membrane, or, if the pore size is too small, the background is not held back. This leads to a restriction in protein sizes that are detectable by the assay.

Another disadvantage of the assay of WO 2008/056165 A1 is the higher likelihood for errors, because, in addition to a typical first step where the aggregates are formed, a second biochemistry step is added for the immobilizing on the trapping zone. This assay also does not provide the potential to use colour multiplexing but needs spatial multiplexing by using different trapping zones. This is disadvantageous because the trapping zone has limited space.

M.A. Fernandez-Baldo et al. (Analyst, 2011,136, pages 2756-2762) [21] presents a microfluidic solution with a competitive immunoassay to test for Ochratoxin A in apples to determine if the fruit is contaminated with Aspergillus ochraceus. Magnetic nanoparticles are used to run the assay automated in 16 minutes. From the automation perspective, the microfluidic system uses pumps to move the liquid in the chip. Due to the use of a pump, an interface with the chip is required, and for this reason, the system itself is not a closed system. This increases the contamination risk. The system also requires several disposables (because cleaning of the interface between pump and chip is difficult for the user) and multiple reagents (which is a disadvantage because all reagents need to be stored on the disposable).

In comparison, the structural format of the method described herein is inherently advantageous and simple (over the named publication) in case of automation because it can be performed as a single-step comprising assay/incubation, followed by separation, no washing but straightforward detection in the bound-free phase all taking place in the same reaction well, therefore minimizing the risk of proteins being lost due to unspecific adsorption in microchannels and chambers. In contrast, the the assay of Fernandez-Baldo et al, when automated on this microfluidic system, requires washing steps.

Because the automation and the fulfillment of immunoassay requirements as outlined in the section "Background of the Invention" strongly depend on the form and complexity of the assay that needs to be integrated - such as the number of different target molecules that need to be simultaneously detected, the number of process steps, components and reactions, and the time of the reaction steps - it is necessary to not only develop a device that fits these requirements, but to take a step back and rethink the biochemical process-chain in order to make the automation as easy and as robust as possible. Accordingly, in light of the prior art there remains a significant need in the art to provide an improved method to be performed in an automated system for detecting a target molecule in a sample.

### SUMMARY OF THE INVENTION

In light of the technical and performance challenges of the prior art, and in relation to the immunoassay requirements outlined in the section "Background of the invention", the technical problem to be solved by the present invention is the provision of alternative and/or improved means for detecting one or more target molecules in a sample in an automated or automated microfluidic system. The problem is solved by the features of the independent claims. Preferred embodiments of the present invention are provided by the dependent claims.

Herein described is a method for detecting a target molecule in a sample, comprising
a. incubating in a buffer solution
   i. the sample suspected to contain one or more target molecules,
   ii. solid phase-coupled capture molecules suitable for binding to the target molecules,
   iii. a defined amount of detection molecules suitable for binding to the target molecules and/or the capture molecules,
b. separating the solid phase-coupled capture molecules from the buffer solution,
c. detecting the remaining detection molecules in the separated buffer solution.

This application also relates to a method performed in an automated system for detecting a target molecule in a sample, comprising
a. incubating in a buffer solution
   i. the sample suspected to contain one or more target molecules,
   ii. solid phase-coupled capture molecules suitable for binding to the target molecules,
   iii. a defined amount of detection molecules suitable for binding to the target molecules and/or the capture molecules,
b. separating the solid phase-coupled capture molecules from the buffer solution,
c. detecting the remaining detection molecules in the separated buffer solution.

The application further relates to a method for detecting a target molecule in a sample, comprising
a. incubating in a buffer solution
   i. the sample suspected to contain one or more target molecules,
   ii. solid phase-coupled capture molecules suitable for binding to the target molecules, wherein the solid phase is a bead,
   iii. a defined amount of detection molecules suitable for binding to the target molecules and/or the capture molecules, wherein the detection molecules are coupled to beads that are differentiable from the beads coupled to the capture molecules, for example by size, density, magnetic properties,
b. separating the solid phase-coupled capture molecules from the buffer solution and potentially the beads coupled to the detection molecules,
c. detecting the remaining bead-coupled detection molecules in the separated buffer solution.

In embodiments of the invention, the separating of the bead-coupled capture molecules can be achieved by sedimentation, wherein the beads coupled to the capture molecules have a defined weight and/or density that is higher than the weight and/or density of the beads coupled to the detection molecules. Furthermore, the bead-coupled capture molecules can be separated since these beads are heavier and/or show either a higher density and/or larger size than the beads coupled to the detection moleculs. In further embodiments, the beads of the bead-coupled capture molecules can be magnetic, enabling magnetic separation.

The method of the present invention is advantageous compared to known heterogeneous ELISA based methods for detection of a target molecule in a liquid sample because it enables detection and optionally quantification of one or more specific target molecules in a potentially complex sample, by analyzing the separated supernatant after performing the binding reaction between the capture molecules, the target molecules and the detection molecules.

For analysis of the supernatant for detecting the remaining detecting molecules in the separated buffer solution after the separating step b., the separated buffer solution and the solid-phase coupled capture molecules can still be in the same reaction well where the incubation and separation took place. In this context, separation refers to the separate location of the detection molecules that are soluble in the liquid phase in contrast to the detection molecules that are bound to the solid phase of the capture molecules after the incubation step. However, this separate location can be in the same reaction well or container.

Accordingly, the term "separating the solid phase coupled capture molecules from the buffer solution" also refers to concentrating or localizing the solid phase coupled capture molecules in a specific location of the reaction well, so that the solid phase coupled capture molecules are not dissolved in the liquid phase. Since incubation of the reaction components occurs in a reaction well, "separating the solid phase coupled capture molecules from the buffer solution" may more clearly indicate that separation occurs while the solid phase coupled capture molecules and the buffer solution comprising unbound detection molecules are still in the same reaction well also be referred to as "separating in the reaction well the solid phase coupled capture molecules from the buffer solution". Accordingly, as disclosed herein separating capture molecules and buffer solution after incubation step is independent from transferring the separated buffer solution to a different reaction well before detection. Detection of the unbound detection molecules in the separated buffer solution can occur either in the same reaction well, or after transfer of a fraction of the separated buffer solution comprising the unbound detection molecule to another reaction well.

In other words, separation does not necessarily refer to removal of the liquid phase/buffer solution, which has been separated from the solid phase coupled capture molecules, from the reaction well after the incubation.

For example, in case the solid phase that is coupled to the capture molecules is the bottom of a well plate, the separation occurs automatically, since the bound detection molecules are no longer localized in the liquid phase. In another case, where the solid-phase is a bead coupled to the capture molecules, the beads may be separated from the buffer solution/liquid phase by concentrating the beads in a specific location of the reaction well, for example by magnetic or centrifugal separation, electrophoresis or sedimentation. After the separating step, the buffer solution/liquid phase may or may not be removed from the reaction well.

Detection of the remaining detection molecules in the separated buffer solution occurs in the same well as the incubation step. In embodiments, the method of the invention is a heterogenous detection method. It was entirely surprising that the method provides reliable results, since heterogeneous detection assays usually require analysis of the solid phase and detection and quantification of the binding of detection molecules on the solid phase for a reliable, robust and reproducible result. Equally surprising is the fact that, the method of the present invention does not require a washing step and, even though the unbound detection molecules can be in the same well as the separated solid phase-coupled capture molecules, it still delivers quantitative results, which was unexpected in view of the state of the art. In embodiments, the invention also relates to a method performed in an automated system for quantitatively detecting a target molecule in a sample.

The method of the present invention is advantageous compared to known heterogeneous ELISA based methods for the detection of a target molecule in a liquid sample because by analyzing the separated supernatant in a competitive format the signal will increase with increasing target concentration, while in a sandwich format the signal will decrease with increasing target concentration. This is advantageous, because especially sandwich assays are often required to be very sensitive and one of the restrictions is the detection limitation of the optical hardware that provides low signals for low concentrations in standard ELISA approaches. However, with the method of the present invention the sensitivity can be enhanced in two ways: (i) by using a particle-based detection the fluorescence signal is enhanced due to the high amount of fluorophores in the core of the detection particle (detection-bead) and (ii) by detecting in the supernatant the lower concentration of the target molecule leads to high signal and therefore makes the assay more sensitive.

However, known methods directed to the analysis of the solid phase require washing of the solid phase, preferentially with a specific washing buffer, to provide reliable results and to avoid falsification of the results by unspecifically bound detection molecules. Such additional washing steps of the solid phase take a lot of extra time and require additional reagents, such as the washing buffer(s). Such washing steps, either if done manually or automatically, pose the additional risk of proteins being lost due to pipetting (in case of manual) or non-specific binding on chambers and challens walls (in case of microfluidically or not automated platforms). Therefore, known methods are disadvantageous for applications that require quick results, which should preferably be carried out in an automated system suitable for the point of care (e.g. compact and portable) and/or by a person with limited laboratory training.

The method of the present invention enables reliable detection and quantification of the presence of at least one specific target molecule in a sample by analyzing the separated unbound phase (separated buffer solution) after co-incubation of the capture, target and detection molecules. This feature is particularly advantageous, since it leads to a drastic reduction of the required method steps for determining the presence of and quantifying a target molecule. In the context of the method of the invention, the separating of the solid phase-coupled capture molecules and the buffer solution refers to separating the buffer solution with the unbound detection molecules (either in whole or with a specified volume) from the solid phase-coupled capture molecules. In the context of the separating step of the method of the present invention, the term buffer solution refers to the liquid phase present during the incubation of the components of the method of the invention, comprising the buffer solution and potentially liquids of the further components of the method of the invention. In the context of the present invention, separating the solid phase-coupled capture molecules from the buffer solution (step b) does not

require removal of the liquid phase or parts thereof from the reaction well comprising the solid-phase coupled capture molecules and potentially bound detection molecules. Instead, these components remain in the same reaction well or container, but in separate locations so that the solid phase coupled capture molecules are not dissolved in the liquid phase.

For example, after the incubation step, the solid-phase is separated from the solid phase-coupled capture molecules but still remains in the same reaction well, and the unbound detection molecules dissolved in the liquid phase are detected.

Here described is a method for detecting a target molecule in a sample, comprising the sequential steps of
a. incubating at the same time in a buffer solution in the same reaction well
   i. the sample suspected to contain one or more target molecules,
   ii. capture molecules suitable for binding to the target molecules, wherein the capture molecules are coupled to beads (capture-beads),
   iii. a defined amount of detection molecules suitable for binding to the target molecules and/or the capture molecules, wherein the detection molecules are coupled to beads (detection-beads),
      wherein the capture-beads are separable from the detection-beads,
b. separating in the reaction well the capture-beads from the buffer solution comprising unbound detection-beads,
c. detecting remaining unbound detection-beads in the buffer solution, which has been separated from the capture-beads.

The method of the present invention uses a two-bead approach. By using not only one but two beads the following can be achieved: (i) a higher surface to volume ratio, enabling more proteins to be bound on the surface. Therefore, during the incubation step, which may involve mixing or agitation of the buffer solution, the interaction probability of the proteins is enhanced and the required incubation time is shortened. (ii) Easy and rapid separation of the capture molecules bound to one of the two kinds of particles can be achieved by exploiting physical principles such as magnetic separation, centrifugation, electrophoresis or other methods known to the person skilled in the art.

In cases where the solid phase that the capture molecules are bound to is a bead, the term capture-beads may be used to refer to the bead-coupled capture molecules. Analogously, in cases where the detection molecules are coupled to beads, the term detection-beads may be used to refer to the bead-coupled capture molecules. An embodiment of the invention that comprises capture-beads and detection-beads may be referred to as two-bead approach, a two-bead variant or a two-bead assay of the method of the invention. In the context of a two-bead approach of the invention, the beads that are coupled to the capture molecules and the beads that are coupled to the detection molecules are distinguishable or differentiable. This means that two kinds of beads should be used for coupling either to the capture molecules or the detection molecules, wherein the kinds of beads have distinguishable properties, such as, without limitation, size, shape, diameter, weight, density, material, magnetic properties, electrophoretic properties, labeling and/or color.

Importantly, in the context of a two-bead approach the capture-beads can be separated from the detection-beads after both kinds of beads have been incubated in the buffer solution, which means that the capture-beads are separable from the detection beads. Separability can be achieved due to the distinbuishable properties of the beads. Furthermore, the capture-beads can be separated/are separable from the buffer solution. Separation of the capture-beads from the buffer solution and also the unbound detection-beads present in the buffer solution after the incubation step occurs in the reaction well of the incubation step.

The term "incubating at the same time" as used herein refers to embodiments of the invention in which the reaction components of step a. (incubating) of the method are not added sequentially to the buffer solution, but at about the same time, so that the binding reactions occur while all components are present in the buffer solution. This means that the method of the invention is carried out as a one-step assay/reaction.

The term "sequential steps" refers to the steps a. (incubating), b. (separating), and c. (detecting) of the method of the invention, wherein sequential means that the steps are performed sequentially in the listed order of first a. , second b. and third c.

In embodiments of the invention, the detection of remaining unbound detection molecules or detection-beads occurs in the same reaction well as the incubation and separation. This represents a great advantage of the method of the invention, since there is no requirement for a second reaction well for detecting the unbound detection molecules, which is particularly advantageous for designing automated systems for performing the method of the invention and for avoiding the risk of protein and/or bead loss during the transfer to a second reaction well for detecting the unbound detection molecules.

Furthermore, due to the incubation of a defined/known amount of detection molecules, the initial concentration of detection molecules in the liquid phase of the incubation step is known and a potential decrease of the concentration of detection molecules after the incubating step can be measured in a separated fraction of the liquid phase and does not require separation of the whole liquid phase from the solid phase. Another advantage is that the present invention does not need a membrane or any other fibrile matrix to operate and it still gives quantitative results on the amount of tested analyte.

In embodiments of the invention involving one kind of beads as a solid phase coupled to the capture moelcules and a different kind of differentiable bead coupled to the detection molecules, it was proven that it is possible to detect very high concentration of a target (15 mg/l - 120 mg/l) without a dilution step for sample preparation. Due to the high surface to volume ratio of the capture-beads and the high amount of capture-molecules on their surface the amount of target that can bind in one reaction is increased. The two-bead approach and the detection of the supernatant leads surprisingly to a very robust assay that is able to withstand any unspecific reaction of the complex sample matrix that is present in a higher proportion if the sample is not diluted. The reduction of the unspecific reaction is uniquely enhanced by the fact that the incubation, the separation and the detection of unbound detection-beads in the unbound phase are all done in the same reaction well. It is surprising that the assay does not show any matrix effects although no washing step is required to conduct the immunoassay. This is a great advantage of the two-bead assay approach that is achieved by coupling the capture and detection molecules to two kinds of beads. The high surface to volume ratio leads to a higher available amount of proteins on the beads' surface. The mass of the beads also leads to a better reaction behavior due to the decreased dependency on the diffusion factor during incubation.

In a preferred embodiment of the invention, the capture-beads and/or the detection-beads are single compact beads. Single compact beads are different from cluster of beads. Such clusters of beads consist of several smaller beads, that are assembled to form a larger bead by forming a cluster or agglomerate of smaller beads. The use of such clusters of beads is disadvantageous in the context of the method of the invention, since the clusters can get destabilized in certain buffers and therefore restrict the selection of the buffer solution. Destabilization and disintegration of the clusters leads to a change of the physical properties of the bead-clusters, changing the separation conditions for the capture-beads and detection-beads and can therefore majorly disturb performance of the method of the invention. Such disturbances cannot occur when the beads used in the context of the invention are single solid beads that are not assembled from smaller bead structures. Accordingly, in preferred embodiments the beads used in the context of the method of the invention are not formed by clusters of beads.

In preferred embodiments of the present invention, the capture-beads have a higher density than the detection-beads. In embodiments, the capture-beads and the detection-beads may have the same or about the same density. In embodiments of the invention, the combination of capture-beads and detection-beads used is such, that the density of the detection-beads is smaller or equal to the density of the capture-beads.

Preferred densities of the capture-beads and/or detection-beads to be used are in the range of about 1 g/ml to 2 g/ml. Possible densities are 1.000, 1.005, 1.01, 1.015, 1.02, 1.025, 1.03, 1.035, 1.04, 1.045, 1.05, 1.055, 1.06, 1.065, 1.07, 1.075, 1.08, 1.085, 1.09, 1.095, 1.10, 1.11, 1.12, 1.13, 1.14, 1.15, 1.16, 1.17, 1.18, 1.19, 1.20, 1.21, 1.22, 1.23, 1.24, 1.25, 1.26, 1.27, 1.28, 1.29, 1.30, 1.31, 1.32, 1.33, 1.34, 1.35, 1.36, 1.37, 1.38, 1.39, 1.40, 1.41, 1.42, 1.43, 1.44, 1.45, 1.46, 1.47, 1.48, 1.49, 1.50, 1.51, 1.52, 1.53, 1.54, 1.55, 1.56, 1.57, 1.58, 1.59, 1.60, 1.61, 1.62, 1.63, 1.64, 1.65, 1.66, 1.67, 1.68, 1.69, 1.70, 1.71, 1.72, 1.73, 1.74, 1.75, 1.76, 1.77, 1.78, 1.79, 1.80, 1.81, 1.82, 1.83, 1.84, 1.85, 1.86, 1.87, 1.88, 1.89, 1.90, 1.91, 1.92, 1.93, 1.94, 1.95, 1.96, 1.97, 1.98, 1.99, 2.00 g/ml.

In further embodiments, the diameter of the capture-beads to be used in the context of the invention is at least 0.5 µm. Further possible diameters of capture-beads comprise the range of 0.5 µm to 5 µm, for example about 0.51, 0.52, 0.53, 0.54, 0.55, 0.56, 0.57, 0.58, 0.59, 0.60, 0.61, 0.62, 0.63, 0.64, 0.65, 0.66, 0.67, 0.68, 0.69, 0.70, 0.71, 0.72, 0.73, 0.74, 0.75, 0.76, 0.77, 0.78, 0.79, 0.80, 0.81, 0.82, 0.83, 0.84, 0.85, 0.86, 0.87, 0.88, 0.89, 0.90, 0.91, 0.92, 0.93, 0.94, 0.95, 0.96, 0.97, 0.98, 0.99, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0 µm.

In an example, the diameter of the detection-beads is at least 0.1 µm. Further possible diameters are in the range of 0.1 µm to 4 µm, for example about 0.11, 0.12, 0.13, 0.14, 0.15, 0.16, 0.17, 0.18, 0.19, 0.20, 0.21, 0.22, 0.23, 0.24, 0.25, 0.26, 0.27, 0.28, 0.29, 0.30, 0.31, 0.32, 0.33, 0.34, 0.35, 0.36, 0.37, 0.38, 0.39, 0.40, 0.41, 0.42, 0.43, 0.44, 0.45, 0.46, 0.47, 0.48, 0.49, 0.50, 0.51, 0.52, 0.53, 0.54, 0.55, 0.56, 0.57, 0.58, 0.59, 0.60, 0.61, 0.62, 0.63, 0.64, 0.65, 0.66, 0.67, 0.68, 0.69, 0.70, 0.71, 0.72, 0.73, 0.74, 0.75, 0.76, 0.77, 0.78, 0.79, 0.80, 0.81, 0.82, 0.83, 0.84, 0.85, 0.86, 0.87, 0.88, 0.89, 0.90, 0.91, 0.92, 0.93, 0.94, 0.95, 0.96, 0.97, 0.98, 0.99, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0 µm.

It is possible to use capture-beads and detection-beads of about the same size, capture-beads with a larger diameter or with a smaller diameter as compared to the detection-beads.

The combination of density and dimensions of the capture- and detection-beads is chosen in such way as to allow a clear separation of the two kinds of beads. For example, but not restricting, one could select similar densities but different dimensions of the capture- and detection-beads; or similar dimensions and different densities. The above ranges are chosen in such way as to retain the advantages of the two-bead based assay indicated herein.

Here described is a method for detecting a target molecule in a sample, comprising the sequential steps of
a. incubating at the same time in a buffer solution in a reaction well
   i. the sample suspected to contain one or more target molecules,
   ii. capture molecules suitable for binding to the target molecules, wherein the capture molecules are coupled to beads (capture-beads),
   iii. a defined amount of detection molecules suitable for binding to the target molecules and/or the capture molecules, wherein the detection molecules are coupled to beads (detection-beads),
      wherein the capture-beads are separable from the detection-beads,
b. separating in the reaction well the capture-beads in the buffer solution comprising unbound detection-beads, and
c. detecting remaining unbound detection-beads in the buffer solution, which has been separated from the capture-beads
wherein the method does not comprise a washing step.

In embodiments, the method of the invention does not comprise a washing step. In particular, the method of the present invention does not comprise washing of the solid phase after incubation with the sample and the other reaction components, and surprisingly still the method provides quantitative results, which makes it advantageous as compared to known assays, such as lateral flow assays. No washing of the solid phase is required after separation of solid phase and liquid supernatant, since the supernatant is analyzed. In particular, it is not even required that the supernatant/buffer solution is transferred to a different reaction well/container after separation. This is not only advantageous with respect to the number of method steps, but also reduces the number of reagents and buffers required for carrying out the method and also the number of reaction wells/reaction containers. This simplifies the performance of the method of the invention by a person with limited training and also reduces the sources for errors. Furthermore, this also simplifies the development of automated systems designed for carrying out the method of the invention in an automated fashion, since such a system has to integrate fewer method steps and fewer reagents as compared to a system designed to carry out a heterogeneous assay of the state of the art that requires analysis of the solid phase. An additional advantage in case of integration in a microfluidic system is that, when the incubation, the separation and the supernatant detection takes place in the same chamber, less microchannels and microchambers are used, therefore less risk for protein loss through unspecific binding on the microfluidic walls.

In a further embodiment of the method of the invention the detected intensity of the signal deriving from the remaining detection molecules in the separated buffer solution is dependent on the concentration of the detection molecules and not on the total amount of the fluorescent label bound to the solid phase, as in the state of the art. Furthermore, in the context of embodiments of the invention, the nature of separation and detection method does not allow the capture beads to interfere with the liquid phase and therefore with the detection taking place in the liquid phase/supernatant. Consequently, the separation process does not have an influence on the test results, and due to the above reasons high reproducibility (low CVs) can be achieved in measurements of the method of the invention. Due to separation of the capture-beads from the liquid phase before detecting remaining detection-molecules in the separated liquid phase, the separation process does not have an influence on the test results, and due to the above reasons high reproducibility (low CVs) can be achieved in measurements of the method of the invention. This simplifies the process of separation and allows the user to separate the solid phase and detect the unbound phase even in the same reaction well without transferring the separated buffer solution to a different container or reaction well. In methods known in the art, where the signal is detected in the solid phase, all beads have to be present to achieve the right result. This means that during separation and all washing steps in such methods it has to be ensured that no capture molecules/capture-beads are removed by accident. In fact, the percentage of bead loss is regarded as an important quality criterium of methods of the state of the art, especially for automated assays. Since the method of the present invention does not rely on detection of the signal of the solid phase, this represents a great advantage over such methods of the state of the art and enables provision of exact and highly reproducible results while reducing the complexity of the method.

The method of the present invention also gives the possibility to not remove any buffer solution from the reaction well of the incubation but instead detect the signal in the liquid phase (supernatant), for example after sedimentation of the beads coupled to the capture molecules in such a way that the detection molecules on their surface are not affecting the detection of detection molecules in the supernatant. Results generated by such embodiments show essentially the same result as results generated by embodiments involving a separation and transfer of the supernatant. In conclusion the immunoassay presented in this invention combines the advantages of the classical approach of a heterogeneous ELISA and the advantages of homogeneous immunoassays.

The development of the method of the present invention, which is preferably carried out as an immunoassay, has been driven by the aim of facilitating automated execution or performance of an immunoassay, for example on a microfluidic platform, preferably an automated centrifugal microfluidic platform. An automated platform and in particular a fully automated centrifugal platform, as well as any other automated and miniaturized platform, does not only aim for the automation of the steps or a method to be performed, but also for integrating the reagent storage into the disposable parts of the system. The challenging demands on such an assay, which are fulfilled by the method of the present invention, but not by known methods of the state of the art, comprise reduction of hands-on time (manual method steps); reduction of the number of required method steps; reduction of the number or required reagents (which may have to be stored in a microfluidic system); simplification of the process-chain operations for an easy automation; prevention of process-related protein loss due to e.g. non-specific binding; reduction of the time for performing an immunoassay; enablement of multiplexing; enablement of performance of sandwich as well as competitive immunoassays (even in parallel and in the same reaction well), to ensure coverage of a broad variety of applications in terms of (i) concentration levels (competitive assays are preferable for detecting and determining analytes present in high concentrations) and (ii) molecular size of the analyte (sandwich assays require analytes with two epitopes to be recognized by two different antibodies, so that small size analytes may require use of a competitive assay); a broad implementation on multiple platforms.

By using two kinds of differentiable particles/beads, one for the solid phase coupled to the capture molecules, and one coulpled to the detection molecules, the stability of the reagents is enhanced. Taking immunoassay as an example, protein stability is a strong challenge for automated systems. Proteins bound to the surface of a bead show long reagent stability in liquid stored in the fridge (2-8°C). The binding of the protein to the surface leads to a stabilizing of the protein due the reduction of mobility, which is a major cause for denaturing processes. The binding of the reagents to the surface of particles also allows the storage of the proteins at room temperature (15-25°C) in disposables for automated systems by drying the particles.

The configuration or design of the method of the invention allows it to be performed either as a sandwich assay or as a competitive assay. It is a great advantage that the method can be performed in a sandwich and a competitive configuration (even in parallel and in the same reaction well), thereby covering a very broad detection range of concentrations as well as molecular sizes and structures of analytes to be detected. Considering that a sandwich assay requires two antibodies that bind to different epitopes of an analyte molecule, this assay configuration can only be performed for suitable target molecules. In addition, it is possible to apply the method of the invention in the competitive configuration. This may be advantageous in case of small analytes that do not comprise two epitopes for which suitable antibodies are available.

The method of the invention can be performed in a standard laboratory setup, either manually or automated, or in a miniaturized platform, which may employ different solid phase platforms and/or different detection methods. In preferred embodiments, the method of the present invention is an immunoassay. The method is advantageous because it is very flexible, in a technical as well as an operational sense.

The method can be implemented in both, standard laboratory equipment, and miniaturized (microfluidic) platforms, for example, but not limited to, microtiter plates and corresponding plate readers, flow cytometers, centrifugal/pressure-driven microfluidics, magnetic-bead-based automated pipetting systems, and generally with any standard or miniaturized system or platform that can make use of a physical principle to separate the bound-free phase/liquid supernatant from the solid phase after co-incubation of the reagents. This flexibility is not possible with assays known in the art, which may require complex setups involving, for example, a membrane for phase separation and subsequent detection, thereby substantially restricting the assay applicability.

Importantly, the method disclosed herein does not require a washing step. In particular, no washing step is required after the separation and before the detection of the unbound phase, even when the latter takes place in the same reaction well that separation was done, which is not trivial for the state of the art, but unique and surprising. That reduces the total number of steps and therefore the time-to-answer, and makes the assay more suitable for an easy automation compared to known methods of the state of the art. Especially in case of implementation of the method of the invention in miniaturized systems, the fact that only one reaction buffer is required for performing the method, while there is no need for further buffers, such as washing buffer or the like, represents an important advantage. Surprisingly, the lack of a washing step in the method of the invention did not have a negative influence on the assay performance and reproducibility of the results, presumably also due to reduced protein loss and reduced unspecific binding as all involved steps (incubation, separation, detection of supernatant) are done in the same reaction well.

The proposed working process comprises the detection of the detection molecules in the liquid phase after co-incubation and separation from the solid phase. The detection molecule referred to in the context of the method of the invention represents a primary detection molecule. Detection can occur either through direct or indirect detection using a label.

In preferred embodiments the detection molecule comprises a label. The label is preferentially attached directly to the primary detection molecule, enabling direct detection. However, it may also be linked to a secondary or tertiary detection molecule, which specifically bind to the primary or secondary detection molecule, respectively, which can be useful for signal amplification in the detection of analytes present in a low concentration. Accordingly, the method here described is compatible with multiple detection principles, such as, without limitation, fluorescence, chemiluminescence, absorption and enzymatic reactions. One of the examples described herein uses fluorescent beads as a label attached to the detection molecule. In embodiments of the invention, the method is carried out as a multiplex assay. The term multiplexing refers to the parallel detection of two or more different target molecules within the same reaction chamber/well, wherein different detection molecules comprising different labels or detection methods are used for each target molecule. This is particularly advantageous for simultaneous detection of multiple markers.

By using one kind of bead as a solid phase coupled to the capture molecules and a differentiable second kind of bead coupled to the detection molecule a shorter reaction time is needed to bind the target molecule, the sample matrix effects are reduced and a high signal-to-nose ratio and an increased sensitivity can be achieved. This is very advantageous for multiplexing and simplifies the process. Therefore, in the context of a multiplex assay using bead-coupled detection-molecules and bead-coupled capture-molecules, for each target molecule to be detected a specific detection-bead has to be used that is distinguishable from the capture-beads and the detection-beads of the other target molecules. Furthermore, all detection-beads have to be separable from the capture-beads.

According to embodiments of the invention, two or more different target molecules comprised in the sample are detected in parallel. In embodiments, the method of the invention can be used to detect two or more target molecules in a sample. In such a multiplex variant of the method, it is possible to detect in parallel different target molecules. Therein, it is possible to detect at least one target molecule of a high concentration in parallel and in one reaction well with at least one other target molecule, which is present in a low concentration.

In particular, due to the above described advantages of the in this invention presented immunoassay method using two kinds of beads, wherein a first kind of bead is coupled as a solid phase to the capture molecules (capture-beads) and a second differentiable kind of bead is coupled to the detection molecules (detection-beads), it is possible to also perform multiplexing of dynamic ranges where one target is present in a range of higher concentrations (e.g. 1000 - 200 000 ng/ml) and would normally be detected with a competitive format, while the second target is present in the same sample matrix in the range of lower concentrations (e.g. 0.001 - 100 ng/ml) and would normally be detected with a sandwich format. Multiplexing of these two targets from one sample and in one liquid is therefore very challenging for standard immunoassay formats. But with the immunoassay method presented in this invention it is possible due to the improved mixing properties and due to the fact that the workflow of a sandwich and a competitive format is conducted with the same steps. Experiments even showed that multiplexing of two targets with different sample matrixes present in detection ranges that differ in 4 to 5 orders of magnitude is possible. This proves that the immunoassay method presented in this invention is surprisingly able to meet the demands even for challenging and complex multiplexing even though no washing step is conducted and even though all incubations, separations and detections of all multiplexed targets are (potentially) performed in the same reaction well or container.

In an aspect, two or more different target molecules comprised in the sample are detected in parallel, wherein
a. a first one target molecule is detected by a sandwich assay and a second target molecule is detected by a competitive assay, or
b. both target molecules are detected by a sandwich assay, or
c. both target molecules are detected by a competitive assay, and
d. one of the target molecules is present in a high concentration and the other target molecule is present in a low concentration, or
e. both target molecules are present in a high concentration, or
f. both target molecules are present in a low concentration.

In embodiments of the invention, two or more different target molecules comprised in the sample are detected in parallel, wherein
a. one or more target molecules are detected by a sandwich assay and one or more other target molecules are detected by a competitive assay, or
b. all target molecules are detected by a sandwich assay, or
c. all target molecules are detected by a competitive assay, or and
d. one or more of the target molecules are present in a high concentration and one or more other target molecules are present in a low concentration, or
e. all target molecules are present in a high concentration, or
f. all target molecules are present in a low concentration.

In an aspect, two or more different target molecules comprised in the sample are detected in parallel, wherein
a. one or more target molecules are detected by a sandwich assay and the other target molecules are detected by a competitive assay, or
b. all target molecules are detected by a sandwich assay, or
c. all target molecules are detected by a competitive assay, or
d. one or more target molecules are dectected by a competitive assay and the other target molecules are detected by a sandwich assay
   and
e. one or more of the target molecules are present in a high concentration and the other target molecules are present in a low concentration, or
f. all target molecules are present in a high concentration, or
g. all target molecules are present in a low concentration, or
h. one or more of the target molecules are present in a low concentration and the other
   target molecules are present in a high concentration.

Such multiplexing assays are preferably performed in a two-bead assay format, using bead-coupled capture- and detection-molecules for each target molecule.

It is an important advantage of the method of the present invention that samples comprising either a high or a low concentration of a target molecule to be detected can be used. The detection range of the assay varies from low over medium to high concentrations. Furthermore, when used as a multiplex assay, it is possible to detect at least one analyte with a high and at least one analyte with a low concentration in the same sample in parallel and in the same reaction well. For example, it is possible to analyze an undiluted sample, for example a serum or plasma sample, which may comprise a high concentration of a certain biomarker and a low concentration of a second biomarker, and both biomarkers could be determined in the same undiluted sample by using the method of the present invention.

In embodiments of the invention no washing step is used. It was proven that the immunoassay of the invention is surprisingly able to robustly detect both low concentrated targets such as in the range of 1 - 2000 ng/ml, and very high concentrated targets, such as in the range of 15 - 120 mg/l. Therefore, in an aspect the concentration of the target molecule in the sample is 1 - 2000 ng/ml. In further examples, the concentration of the target molecule is 15 - 120 mg/l.

The assay here described provides quantitative results for detection of one or more target molecules, preferably in a concentration range of 1 ng/ml to 120 mg/l. in an aspect, the assay provides quantitative results for detection of one or more target molecules present in a high concentration. As here described, the assay provides quantitative results for detection of one or more target molecules present in a low concentration. The term quantitative resuls for detection of one or more target molecules refers to a detection of a target molecule that does not only detect the presence of the target molecule, but also the amount of concentration of the molecule in a sample, for example as compared to one or more reference samples.

A high concentration may relate to a concentration of an analyte, preferably a protein or peptide, of over 10, 20, 30, 40, 50, 60, 70, 80, 90, 100, 110, 120, 130, 140, 150, 160, 170, 180, 190, 200, 220, 240, 260, 280 or 300 mg/l. A high detection range may refer to a range of 10-300, 15-250, 20-200, 24-180, 28-160, 30-140, 32-120, 35-100, 40-95, 45-90, 50-85, 55-80, 60-75, 65-70 mg/l.

A low concentration may relate to a concentration of an analyte, preferably a protein or peptide, of below 2000, 1500, 1000, 800, 600, 500, 400, 300, 200, 100, 90, 80, 70, 60, 50, 45, 40, 35, 30, 25, 20, 18, 16, 14, 12, 10, 9, 8, 7, 6, 5, 4, 3, 2, 1, 0.5 , 0.1 or 0.05 ng/ml. A low detection range may refer to a range of 0.05-10, 0.1-10, 1-2000, 2-1500, 3-1000, 4-800, 5-600, 6-500, 8-400, 10-300, 12-200, 14-150, 16-140, 18-120, 20-100, 25-95, 30-90, 35-85, 40-80, 45-75, 50-70, 55-65 ng/ml.

The possibility to achieve quantitative results for the concentration of a target molecule in a sample over such broad concentration ranges and even without washing step in the workflow of the herein invention is particular possible for embodiments of the invention that use a two-bead approach. Therein, it is particularly advantageous to use capture-beads with a high densitity and large dimensions because it plays a key role in the mixing behavior during incubation with the target molecules and the detection beads/molecules, and because of the post-incubation clear separation of the capture-beads, which subsequently leads to reproducible complexes of solid phase-analyte-detection particle. Preferable (but not restrictive) bead features enabling these functional features are for the capture beads diameter between 0.5 µm - 5.0 µm, densities > 1 g/ml and if needed additional properties like magnetic, electrophoretic or others; for the detection-bead features are bead diameter between 0.1 µm - 4 µm, densities lower or equal than the capture-bead and labels or properties that enable the detection of the bound free detection-beads after separation. The combination of density and dimensions of the capture- and detection-beads should be chosen in such way as to allow a clear separation of the two.

According to embodiments of the invention, the method does not require more than one buffer solution. Preferably, the only buffer solution that is required in the context of the present invention is the buffer used for the incubation of the solid phase-coupled capture molecules, the sample and the detection molecules. Therein, the three components of step a. of the method of the invention may be added to and incubated in the buffer solution simultaneously.

Alternatively, the sample and the capture molecules may be incubated first without the detection molecules, and the detection molecules may be added subsequently. However, also in case of sequential incubation, only one buffer solution is needed.

The detection of the detection molecules in the separated liquid buffer after co-incubation of the components of the method of the invention may require additional buffer solutions, depending on the method of detection. For example, enzyme detection may require additional buffer solutions. However, in embodiments of the invention, for the steps of incubating the reaction components and separating the liquid from the solid phase only one buffer solution is required.

In embodiments, the buffer solution may be provided first. Then the sample, the solid phase-coupled capture molecules and the detection molecules may be added to the buffer solution. Each of these three components may be provided in solid form or in liquid form. One or more of the three components can also be suspended in the buffer solution individually before co-incubation with one or both of the other components. Preferably, the sample suspected to contain one or more target molecules and the detection molecules are added to the solid phase-coupled capture molecules at the same time. The method may be conducted as a one-step assay. In a one-step assay the sample, comprising the target molecules, and the detection molecules are added to the capture molecules in the buffer solution at the same time. Such one-step assays do not involve sequential incubation of reagents, for example a first incubation period, wherein the capture molecules and the target molecules are first incubated in a buffer solution before addition of the detection molecules.

The term "at the same time" is to be understood to mean about at the same time, which relates to the addition of the reagents within a short time interval. Such an interval may be in the range of milliseconds to a few seconds up to one or two minutes, depending on the properties of the reagents and the assay setup. A skilled person is able to judge the meaning of "at the same time" or "about the same time" in the context of the present invention and a specific detection reaction to be performed.

In embodiments of the present invention, the automated system is a microfluidic system. In preferred embodiments, the microfluidic system is a centrifugal microfluidic system. The use of a centrifugal system is particularly advantageous for the method of the present invention since centrifugal forces are well suited for separating the bound-free phase/supernatant from the solid phase-coupled capture molecules comprising the bound target molecules and/or capture molecules. The configuration and principles of centrifugal microfluidics allow the handling of liquids without the need of external pumps or valves, thereby simplifying the cartridge processing equipment. Furthermore, all reagents needed to perform the assay can be stored on the disposable and the assay can be performed in a fully automated fashion with the only hands-on step being the addition of the sample to the system. Additionally, it is possible to perform the sample preparation in an automated way.

In embodiments of the invention, the incubation of the sample, the solid phase-coupled capture molecules and the detection molecules last for no more than 20 minutes. In an example, the method allows an incubation time of only 15 minutes and even less.

However, the incubation time is influenced by different parameters. For example, the time required for incubation of the solid phase-coupled capture molecules with the target molecules and/or detection molecules depends on the specific binding properties and characteristics of the respective molecules to be used. However, the method is preferably carried out with reagents that enable efficient and rapid binding of the reagents to each other if present in the same liquid buffer in solution. In an example, the incubation lasts no more than 30, 28, 26, 24, 22, 20, 19, 18, 17, 16, 15, 14, 13, 12, 11, 10, 9, 8, 7, 6, 5, 4, 3, 2, 1 minutes. Furthermore, the reaction temperature may influence the incubation time. Different incubation times at 37 °C were tested and the assay seemed to be working well also at durations of around 2 minutes, but 15 minutes increases the robustness. In an example, the buffer solution is mixed or agitated during the incubation step.

The fact that the method of the invention can be performed with such short incubation times, which are much shorter than incubation times of known assays, is in particular due to the structural feature of the method employing a two-bead assay principle. This offers an important advantage and has a major impact on the overall time-to-result, and subsequently the time-to-diagnosis and treatment. In embodiments of the invention the solid phase used for coupling the capture molecules is a bead and/or the label of the detection molecule is a bead. Bead coupling of the capture molecule and/or the detection molecule have surprisingly been shown to accelerate the binding reaction during the incubation step. Therefore, setups of the method of the invention wherein one or preferably both of the capture molecule and the detection molecule are bead coupled have been shown to be advantageous since the incubation time can be significantly reduced.

Furthermore, in embodiments of the method of the invention the solid phase is a bead, such as for example a microbead, a nanobead, a charged bead or a magnetic bead. In the context of the invention, a bead that is coupled to the capture molecule is also called a capture-bead. The kind of bead that is used as a solid phase coupled to the capture molecules should preferably have a diameter of more than 0.5 µm to achieve the advantageous properties for the assay as described herein.

The use of capture-beads as a solid phase for coupling the capture molecules is advantageous in the context of the present invention, since beads allow several efficient ways of separating the solid phase-coupled capture molecules with the bound target and/or detection molecules after incubation from the bound-free phase. For example, separation by centrifugation is possible. For example, in case of centrifugal separation, the beads do not have to be magnetic, but only need to have sufficient density to interact with the centrifugal forces generated by the centrifugation. In case of magnetic beads, magnetic removal of the beads from the liquid solution is also possible. In preferred embodiments, separation of the capture beads from the buffer solution occurs through sedimentation. Sedimentation refers to separation through gravitation forces without a need for externally applying further stimuli or forces.

In the context of a two-bead approach, the advantages of using capture-beads are maintained since the capture-beads are seperable from the detection-beads and have different properties that can enable, for example, separation of the capture beads from the buffer solution while the unbound detection-bead remain dissolved in the buffer solution.

In an aspect, separation occurs in no more than 1 minute. By using capture-beads that are larger, heavier and/or have a higher density, preferably beads having a diameter of more than 0.5 µm, in contrast to detection-beads that are smaller, lighter and/or have a lower density than the capture-beads, sedimentation-based separation is possible. This is very advantageous because a magnetic separation as known by the skilled user of the art needs a separation time of at least 2 minutes or longer and additionally requires specific magnetic components for the separation. In contrast, sedimentation-based separation can be achieved in time frames of less than 1 minute, such as for example about 55, 50, 45, 40,35, 30, 25, 20,15, 10 or even 5 seconds. Time to answer/time to get a result from the method of the invention is very important for diagnostic applications in near patient or point-of-care environments. Due to the two-bead approach of this invention not only the incubation time can be reduced strongly but also the separation is simplified and faster. In embodiments of the invention, separating the solid phase-coupled capture molecules from the buffer solution occurs through magnetic, gravitational, centrifugal or electrophoretic forces.

Further advantages of using beads as a solid phase in the context of the present invention include shortened incubation times, easier reagent storage and simplified integration of the method in a microfluidic system. Beads can be easily dispensed and stored on disposables with state-of-the-art high throughput devices (e.g. pipetting roboter). Furthermore, it is possible to prepare a single high volume batch of beads and implement different assays in one disposable without automatization of reagent preparation, which is in particular advantageous for low assay throughput and when the assay is self prepared, for example in research facilities.

In an aspect, a high amount of solid phase-coupled capture molecules is used to ensure that practically all target molecules present in the sample are captured, even if the concentration of the target molecules in the sample is very high. In such aspects, it is advantageous to use beads as solid phase, since this enables to provide a very high solid phase surface-to-volume ratio to couple capture molecules, which increases the number of binding sites on the solid phase. This also enables the detection of high measurement ranges without a dilution of the sample material, which is a big advantage for automated systems as the sample preparation is simplified (and furthermore, advantageous for microfluidic and/or centrifugal microfluidic systems as the footprint of the cartridge is smaller and simpler).

In embodiments not part of the invention the solid phase is a microtiter plate. This embodiment is particularly advantageous for performing the method of the invention manually or by an automated pipetting robot in a multiwell plate. In further embodiments, the solid phase may be a surface of a microfluidic system, such as the surface of a reaction chamber of a centrifugal disk.

In certain embodiments of the invention, the capture molecule recognizes a first epitope comprised by the target molecule. This may be the case for both a competitive as well as a sandwich type assay.

In further embodiments, the detection molecule recognizes a second epitope comprised by the target molecule. This feature refers to sandwich assays, wherein the detection molecule and the capture molecule both bind to the target molecule, but to different epitopes. According to embodiments of the invention, the capture molecule recognizes a first epitope comprised by the target molecule and the detection molecule recognizes a second epitope comprised by the target molecule.

In embodiments of the invention,
a. the capture molecule recognizes a first epitope comprised by the target molecule, and
b. the detection molecule recognizes a second epitope comprised by the target molecule (sandwich assay) or
c. the detection molecule comprises the first epitope recognized by the capture molecule (competitive assay).

In an aspect, the detection molecule comprises the first epitope recognized by the capture molecule. In a variant of the method the capture molecule recognizes a first epitope comprised by the target molecule and the detection molecule also comprises the first epitope recognized by the capture molecule. According to this aspect the first epitope of the detection molecule and the first epitope of the target molecule can both bind to the capture molecule and are therefore competing for binding to the capture molecule. The capture molecule may not be able to bind to two epitopes but can only bind to one epitope.

This relates to competitive assays, wherein the detection molecule and the target molecule compete for binding to the capture molecule. In certain examples the target molecule and the detection molecule are the same molecule, which may differ in certain aspects required for detecting of the detection molecule. For example the detection molecule may be labeled and/or comprise a tag that is recognized by a secondary detection molecule. Alternatively, the detection molecule and the target molecule may only have a certain domain or structure in common, which comprises the epitope recognized by the capture molecule.

Therefore, depending on the abundance or concentration of the target molecules in the incubation buffer, a higher or lower amount of detection molecules will bind to the capture molecules upon simultaneous incubation of the capture molecules, detection molecules and target molecules and will not be present in the buffer solution after separation from the solid phase comprising the coupled capture molecules with bound detection and target molecules. In case of a high concentration of target molecules, the number of detectable detection molecules in the buffer solution after separation will reduce less strongly as compared to a situation with few target molecules. If there is a high concentration of target molecules comprising the first epitope, a lot of capture molecules will bind to a target molecules and are not available for binding to detection molecules. Accordingly, the number of detection molecules after separation from the solid phase will not reduce as strongly, as compared to a situation where there are few target molecules, and all or most capture molecules will then bind to the detection molecules comprising the first epitope, which accordingly will no longer be present in the buffer solution after separation from the solid phase-coupled capture molecules.

In a further embodiment of the invention, the capture molecule comprises an antibody or fragments thereof or an antigen, and/or the detection molecule comprises an antibody or fragments thereof or an antigen. According to a further embodiment, the capture molecule comprises an antibody or fragments thereof and/or the detection molecule comprises an antibody or fragments thereof. Preferably, the method of the invention is an immunoassay comprising at least one antibody and at least one antigen as capture molecule, target molecule and detection molecule.

In preferred embodiments of the invention, the detection molecules are coupled to beads (detection-beads). Therein, the beads may serve as a label. This is advantageous as the beads have a high surface-to-volume ratio, thereby facilitating high number of detection molecules on their surface, which decreases the time-to-interaction between capture molecules and detection molecules. Furthermore, if the beads are fluorescent beads they can be used to amplify the signal as they contain a high number of fluorophore, which increases the signal intensity and thereby the sensitivity of the system. In embodiments of the invention, the capture molecules and the detection molecules are both coupled to beads, wherein the beads coupled to the capture molecules are distinguishable from the beads coupled to the detection molecules. For example, the beads of the capture molecules and the beads of the detection molecules may be of different mass, diameter, size, material, shape, electric properties and/or magnetic properties, and/or may be labeled differently, for example by different fluorescent labels or tags. Preferably, the weight and/or the size and/or density of the kind of detection-bead is less than the weight and/or the size and/or density of the capture-beads.

Here described is a kit for detecting a target molecule in a sample according to the method of any of the present invention, wherein the kit comprises
a. solid phase-coupled capture molecules suitable for binding to the target molecules,
b. detection molecules suitable for binding to the target molecules and/or the capture molecules,
c. and optionally means for detecting the detection molecules in a buffer solution after
   i. incubating in the buffer solution the solid phase-coupled capture molecules, the sample suspected to contain one or more target molecules and a defined amount of the detection molecules, and
   ii. separating the solid phase-coupled capture molecules from the buffer solution.

Preferably, the kit additionally comprises a microfluidic system, such as a centrifugal microfluidic system. Furthermore, the kit may comprise a defined amount of the detection molecules. The components of the kit have been described in the context of the method of the invention. Furthermore, additional means for detecting the detection molecules in a buffer solution are known to the person skilled in the art.

Preferably, the kit comprises
a. capture-beads suitable for binding to the target molecules,
b. detection-beads suitable for binding to the target molecules and/or the capture molecules,
   wherein capture-beads and the detection-beads are single compact beads, the capture-beads are separable from the detection-beads, the capture-beads have a higher density than or equal density to the detection-beads and the capture-beads have a diameter of at least 0.5 µm,
c. optionally a microfluidic system, preferably a centrifugal microfluidic system,
d. and optionally a buffer solution and/or means for detecting the detection-beads in a buffer solution after
   i. incubating in the buffer solution the solid phase-coupled capture molecules, the sample suspected to contain one or more target molecules and a defined amount of the detection molecules, and
   ii. separating in the reaction well the capture-beads from the buffer solution comprising unbound detection-bead.

### DETAILED DESCRIPTION OF THE INVENTION

The invention is defined in the appended claims.

In the context of the present invention, the term "automated system" refers to a system that is able to carry out all (or some of the) necessary steps for detecting one or more target molecules in a sample without the involvement of manual steps by trained personnel after the sample is added to the system. In the context of the invention, the term "automated system" refers to fully automated as well as to (semi)automated systems.

In some (semi)automated cases, it may be that the system requires manual adding of liquids that are necessary for carrying out the steps for detecting the target molecule.

An automated system does not require supervision by trained personnel, thereby the overall labor costs are reduced. Furthermore, manual errors (even by trained personnel) can be avoided and reproducibility of results can be enhanced. In addition, an automated system reduces the hands-on time from sample adding to result compared to the manual workflow.

Importantly, an automated system can be used to transfer a workflow from a trained user to an untrained or limited trained person. Thus, whole biological processes, such as sample preparation, nucleic acid extraction and purification, protein extraction and purification, amplification and detection can be integrated in automated systems and simplified for the end-users, offering advantages such as high-throughput, multiplexing and high parallelization of assays, portability of devices for point-of-care applications, and accurate measurement.

Some processing steps typically performed in automated systems are, for example but are not limited to, dilution of sample material, mixing of the sample with various reagents like capture molecules, detection molecules and buffer solution, washing steps and detection (or preparation for detection).

An automated system may be elaborated so that the incorporated automation allows the user to generate multi-step reactions requiring a low level of expertise and still achieving several functionalities. The automated systems, especially when combined with microfluidic cartridges, can become small in footprint and are able to provide analysis at the point of care / point of need.

In general, automated systems are able to direct, mix, separate or manipulate fluids to attain for example multiplexing, fast results, and high-throughput systems. Various automated systems for immunoassays are known in the art and could be used for performing the method of the present invention, potentially in a modified form. The skilled person is aware of how to use and adjust such systems for carrying out the method of the invention.

Microfluidic devices exploit the physical and chemical properties of liquids and gases at the microscale. An automated microfluidic system in the sense of the present invention relates to a system, which enables precise control and manipulation of fluids that are geometrically constrained to a small, often sub-millimeter, scale. Automated microfluidic systems can be used to move, mix, separate or otherwise process liquids in order to achieve fast reactions, multiplexing, automation, and high-throughput analysis of liquids and preferably target molecules present in the liquids. Microfluidic systems typically fulfill at least one of the features of small volumes (ml, µL, nL, pL, fL), small size, low energy consumption and effects of the micro domain. Numerous applications employ passive fluid control techniques like capillary forces. In some applications, external actuation means are additionally used for a directed transport of the media and liquids. For example, rotary drives/centrifugal microfluidic systems are applying centrifugal forces for the fluid transport on passive chips. Active microfluidics refers to the defined manipulation of the working fluid by active (micro) components such as micropumps or microvalves. Micropumps supply fluids in a continuous manner or are used for dosing. Microvalves determine the flow direction or the mode of movement of pumped liquids. Processes which are normally carried out in a laboratory are miniaturized on a single cartridge in order to enhance efficiency and mobility as well as reduce sample and reagent volumes. Automation in the context of microfluidic systems refers to the fact that, upon initial loading of the system with the required components such as assay buffers, reagents and one or more samples, the assay steps may be carried out automatically without the requirement for further manual interference of the user.

Automated microfluidic systems of the present invention comprise microfluidic chips or cartridges, which are formed by a pattern of microchannels and chambers and eventually other components, which can be molded, thermoformed, engraved or produced by other suitable methods. Some systems need a link to the macro-environment by several holes of different dimensions hollowed out through the chip. It is through these pathways that fluids can be injected into and evacuated from a microfluidic chip or system. In general, microfluidic devices are able to direct, mix, separate or manipulate fluids to attain for example multiplexing, automation, and high-throughput analysis. The microchannels network design must be precisely elaborated to achieve the desired features (lab-on-a-chip, *in situ* sample treatment/preparation, detection of microorganisms, nuclic acids, proteins, or other (bio)molecules, etc.). To accurately manage fluids inside the microchannels, specific systems are required. These elements can be found embedded inside the microfluidic chip, disk, or cartridge.

Microfluidic systems offer several benefits over conventionally sized systems. They allow the analysis and use of less volume of samples, chemicals and reagents reducing the overall manufacturing and application costs and they open new possible areas of application. Many operations can be executed at the same time thanks to the compact size of the microfluidic cartridges, thus increasing the analytical throughput. The micro/nanoscale dimensions allow faster reaction times, thereby shortening the time to result. Microfluidic systems have the capacity to both process and analyze samples with minor sample handling. A microfluidic cartridge may be elaborated so that the incorporated automation allows the user to generate multi-step reactions requiring a low level of expertise and still achieving a lot of functionalities. Such microfluidic systems execute functions that extend from detecting target molecules, such as proteins or toxins, to analyzing DNA sequences or creating inkjet printing devices. Microfluidic systems have diverse assets: fast reaction time, enhanced analytical sensitivity, enhanced temperature control, portability, easier automation and parallelization, integration of laboratory routines in one device (lab-on-a-chip). Microfluidic systems are cheap as they do not involve the use of various costly equipment or devices and reduce the hands-on time, thereby reducing the labor costs. Whole biological process, such as sample preparation, nucleic acid extraction and purification, protein extraction and purification, amplification and detection can be integrated in microfluidics and be simplified for the end-users. These offer advantages such as high-throughput, multiplexed and highly paralleled assays, faster analyses due to the shorter reaction and/or separation times, realization in portable devices for point-of-care applications, low reagent consumption, and accurate measurement. Overall, microfluidics allow to increase the measurement resolution in given applications.

Microfluidic systems comprise so called lab-on-a-chip (LOAC) devices. A LOAC is a device performing on a miniaturized scale one or several analyses commonly carried out in a laboratory. It integrates and automates multiple high-resolution laboratory techniques such as synthesis and analysis of chemicals or fluid testing into a system that fits on a chip. There are many advantages to operating at this scale. Samples analysis can occur on site (point-of-care / point-of-need), where the samples are generated, rather than being carried to a laboratory facility. At the microscale it is easier to control the fluids behavior, the movement and interaction of samples, causing reactions to be much more potent, and minimizing chemical waste. It also allows reduced exposure to dangerous chemicals.

Further examples of automated microfluidic systems known to the person skilled in the art that may be used in the context of the present invention comprise continuous flow microfluidics, digital microfluidics and optofluidic systems.

Preferably, the method of the present invention is carried out in the context of a centrifugal microfluidic system. A centrifugal microfluidic system or a centrifugal microfluidic cartridge is a type of lab-on-a-chip technology that can be used to integrate processes such as separating, mixing, biomolecular reactions and detecting molecules in a single piece of platform, including a disk-shaped platform. This cartridge is an integration of multiple technologies in different areas. Some basic element components such as valves, mixing units, and separating units should all be used to complete the full testing process. There are many methods proposed by scientists which have been proposed to be performed in centrifugal microfluidic systems, including immunoassays. The final step can be receiving data from the cartridge by means of a dedicated processing device. Various centrifugal microfluidic systems are known in the art and could be used for performing the method of the present invention, potentially in a modified form. The skilled person is aware of how to use and adjust such systems for carrying out the method of the invention. Recent developments in the field of centrifugal microfluidics are also disclosed in [19], [22].

In the context of the present invention, the term "target molecule" refers to a molecule of interest to be detected in a sample. Preferably, the target molecule is a biomolecule, such as, for example, nucleic acids, proteins, enzymes, peptides, antibodies, sugar molecules, lipids and combinations thereof, such as, for example, glycosylated proteins or other glycosylated biomolecules.

The term "sample" in the sense of the present invention relates to any kind of liquid or liquefiable material that is suspected to comprise a target molecule. Sample material used in the present invention can be, for example, a biological sample, such as a body fluid or tissue obtained from an organism, such as a mammal or preferably a human, wherein a body fluid or tissue may be blood, serum, plasma, cerebrospinal fluid, urine, saliva, sputum, pleural effusions, a tissue sample, a tissue biopsy, a stool sample, cells, a cellular extract, cells from cell culture, cell culture extracts or cell culture supernatants, and the like. Samples can be also of non-human nature, for example: from the environmental sector: water from basins, ballast water, sea water, and the like; from the food and beverage sector: milk, juice, wine, and the like. Such samples may be analyzed undiluted or diluted and/or after liquefaction.

In the context of the present invention, a sample may be analyzed by the method described herein to determine whether a specific target molecule is present in the said sample or not. In general, a sample that is suspected to contain the target molecule of interest will be analyzed and the method of the present invention may be used to determine the amount and/or concentration of the target molecule in said sample in a quantitative or semiquantitative way, for example in comparison to one or more reference samples, which are known to not contain the target molecule or to contain the target molecule. Preferably, in such a reference sample the exact concentration of the target molecule is known.

As used herein, a "capture molecule" may be any molecule known to interact with or known to bind to the respective target molecule. For example, if the target molecule is a specific antigen, such as a specific biomolecule or protein (cytokine, toxin, proteinaceous biomarker, enzyme, hormone or fragment thereof) the capture molecule can be an antibody. Alternatively, if the target molecule is a specific antibody, the capture molecule may be an antigen known to bind to the specific antibody of interest. The target molecule may be a ligand to a receptor and the capture molecule may be the corresponding receptor, or the other way around. The target molecule could be a nucleic acid molecule of a specific sequence and the capture molecule may be a nucleic acid molecule comprising a sequence that is complementary to the sequence of the target molecule, enabling binding of the target molecule to the capture molecule.

An antibody, also known as an immunoglobulin (Ig), is a large, Y-shaped protein produced mainly by plasma cells that is used by the immune system to bind to specific molecules which are recognized as foreign by the organism, for example molecules of an invading pathogen. The antibody recognizes a target, called an antigen, via the Fab's variable region. Each tip of the "Y" of an antibody contains a paratope that is specific for one particular epitope on an antigen, allowing these two structures to bind together with precision. Using this binding mechanism, an antibody can bind to the corresponding antigen. Antibodies can come in different varieties known as isotypes or classes, such as in placental mammals the five antibody isotypes known as IgA, IgD, IgE, IgG, and IgM, which can all be used in the context of the present invention. Also antibody fragments or specific parts can be used in the context of the present invention. The arms of the Y structure of an antibody contain the sites that can bind to antigens and this region of the antibody is called the Fab (fragment, antigen-binding) region. It is composed of one constant and one variable domain from each heavy and light chain of the antibody. The paratope is shaped at the amino terminal end of the antibody monomer by the variable domains from the heavy and light chains. The variable domain is also referred to as the FV region and is important for binding to antigens. To be specific, variable loops of β-strands, each on the (variable) light (VL) and (variable) heavy (VH) chains are responsible for binding to the antigen. These loops are referred to as the complementarity determining regions (CDRs). The base of the Y is called the Fc (Fragment, crystallizable) region, and is composed of two heavy chains that contribute two or three constant domains depending on the class of the antibody. The Fc region binds to a specific class of Fc receptors, and other immune molecules, such as complement proteins. Monoclonal antibodies are identical antibodies that all have identical paratopes and react with or bind to the same specific epitope of an antigen. In contrast, the term "polyclonal antibody" refers to a group of different antibodies that react with different epitopes of the same antigen. As used herein, the term antibody refers to antibodies and fragments thereof or modified antibody molecules that comprise the antigen-recognition function of antibodies as described above.

An epitope is an antigenic determinant, which is the part of an antigen that is recognized by the immune system, specifically by antibodies or other receptors of the immune system. In the context of the present invention the term "epitope" is used in a broader sense to denominate the structure of a target molecule that is recognized and bound by the capture molecule and/or the detection molecule used in the method of the present invention. Accordingly, the term "epitope" in the sense of the present invention is not limited to a structure or part of an antigen that is recognized by an antibody, but also relates to a specific structure, part or surface area of any target molecule in the sense of the present invention, which is bound by or is interacting with the corresponding capture molecule and/or detection molecule used in the method of the invention.

Detection of a target molecule by the method of the present invention refers to either indirect or direct determination of the presence of the target molecule by means of a detection molecule. A detection molecule is a molecule that is either a labeled molecule, which can be detected directly by means of the label, or can be detected indirectly for example by means of a labeled secondary detection molecule that binds to the (primary) detection molecule. A detection molecule in the sense of the present invention can bind to the capture molecule and/or to the target molecule. In case of binding of the capture molecule to the detection molecule, the method of the present invention is carried out as a competitive assay, wherein the detection molecule and the target molecule are competing for binding to the capture molecule. In case of binding of the detection molecule to the target molecule in the context of the present invention, the method is preferentially carried out as a sandwich type assay, wherein the target molecule binds to both the capture molecule as well as the detection molecule through two different epitopes.

As explained herein, the method of the present invention can be carried out as an immunoassay, involving the binding between antibodies and antigens for the detection of either a specific antigen or specific antibodies in a sample of interest. However, the principles for detecting the presence of a captured target molecule with the aid of a detection molecule employed by immunoassays, which are explained in the following, also apply correspondingly to alternative embodiments of the method of the invention that do not involve antibody-antigen interaction, but involve other target- and capture molecules, such as nucleic acid molecules, receptors and receptor-agonists and further examples disclosed herein and known to the person skilled in the art.

As used herein, an immunoassay is a method that is used for example in laboratory medicine, wherein a specific antibody-antigen reaction is used to detect an analyte or target molecule in a sample. An immunoassay is a biochemical test that measures the presence or concentration of a macromolecule or a small molecule in a solution through the use of an antibody or an antigen. The molecule detected by the immunoassay is often referred to as an "analyte" and is in many cases a protein, although it may be other kinds of molecules, of different size and types, as long as antibodies or other capture molecules that have the adequate properties for binding to the analyte are available. The use of a calibrator is often employed in immunoassays. Calibrators are solutions that are known to contain the analyte in question, and the concentration of that analyte is generally known. Comparison of an assay's response to a real sample against the assay's response produced by the calibrators makes it possible to interpret the signal strength in terms of the presence or concentration of analyte in the sample. Calibrators do not have to be included in every run but can be measured batch wise. In preferred embodiment the method of the present invention is an immunoassay that employs antibodies and antigens as capture molecules and target molecules, or the other way around.

Immunoassays rely on the ability of an antibody to recognize and bind a specific antigen, such as a specific macromolecule, in what might be a complex mixture of molecules or macromolecules, such as a biological sample. In some cases, an immunoassay may use an antigen to detect the presence of antibodies, which recognize that antigen, in a solution. Accordingly, the analyte can be an antigen or an antibody. In addition to the binding of an antibody to its antigen, the other key feature of all immunoassays is a means to produce a measurable signal in response to the binding of the antigen to the antibody, which in the context of the present invention in general corresponds to the binding of the capture molecule to the target molecule. Most, though not all, immunoassays involve chemically linking antibodies or antigens with some kind of detectable label, wherein these labeled antibodies or antigens serve as a detection molecule.

A large number of labels exist in modern immunoassays, and they allow for detection through different means. Many labels are detectable because they either emit radiation, produce a color change in a solution, fluoresce under light, or can be induced to emit light. Suitable labels are known to the skilled person. A non exhaustive list of possible labels comprises, without limitation, enzymes, such as horseradish peroxidase (HRP), alkaline phosphatase (AP) or glucose oxidase; radioactive isotopes; DNA reporters that can be used in a real-time immunoquantitative PCR (iqPCR), wherein the label is used as a DNA probe; fluorogenic reporters; electrochemical tags or labels, which emit detectable light in response to electric current; beads or particles, which may have a specific size and/or weight, and/or which may comprise further labels as described herein (fluorescent label; enzyme label, etc.). Quantum dots or similar inorganic particles represent another possible kind of label to be used in the context of the invention.

Direct labels include fluorescent or luminescent tags, metals, dyes, radio nucleotides, and the like, attached to the detection molecule. A detection molecule such as an antibody labeled with iodine-125 (¹²⁵I) can be used for determining the levels of one or more markers in a sample. A chemiluminescence assay using a chemiluminescent antibody specific for the marker is suitable for sensitive, non-radioactive detection of marker levels. A detection molecule labeled with fluorochrome is also suitable for determining the levels of one or more markers in a sample. Examples of fluorochromes (or fluorophores), that can be used in the context of the present invention comprise, without limitation, rhodamine and derivatives, such as Texas Red, fluorescein and derivatives, such as 5-bromomethyl fluorescein, Lucifer Yellow, IAEDANS, 7-Me2N-coumarin-4-acetate, 7-OH-4-CH3-coumarin-3-acetate, 7-NH2-4CH3-coumarin-3-acetate (AMCA), monobromobimane, pyrene trisulfonates, such as Cascade Blue, and monobromotrimethyl-ammoniobimane, FAM, TET, CAL Fluor Gold 540, HEX, JOE, VIC, CAL Fluor Orange 560, Cy3, NED, Quasar 570, Oyster 556, TMR, CAL Fluor Red 590, ROX, LC red 610, CAL Fluor Red 610, Texas red, LC red 610, CAL Fluor Red 610, LC red 640, CAL Fluor Red 635, Cy5, LC red 670, Quasar 670, Oyster 645, LC red 705, Cy5.5, BODIPY FL, Oregon Green 488, Rhodamine Green, Oregon Green 514, Cal Gold, BODIPY R6Gj, Yakima Yellow, Cal Orange, BODIPY TMR-X, Quasar-570 /Cy3, TAMRA, Rhodamine Red-X, Rhodamine B, Rhodamine 6G, Redmond Red, BODIPY 581/591, Cy3.5, Cal Red/Texas Red, BODIPY TR-X, BODIPY 630/665-X, Pulsar-650, Quasar-670/Cy5. Fluorescent labeling can also occur through fluorescent proteins, such as, for example, photo proteins, such as aequorin; obelin; Aequorea fluorescent proteins, such, e.g., green fluorescent proteins (GFP, EGFP, AcGFP1), cyan fluorescent proteins (CFP, ECFP), blue fluorescent proteins (BFP, EBFP), red fluorescent proteins (RFP), yellow fluorescent proteins (YFP, EYFP), ultraviolet fluorescent protein (GFPuv), their fluorescence-enhancement variants, their peptide destabilization variants, and the like; coral reef fluorescent proteins, such, e.g., Discosoma red fluorescent proteins (DsRed, DsRed1, DsRed2, and DsRed-Express), Anemonia red fluorescent proteins (AsRed and AsRed2), Heteractis far-red fluorescent proteins (HcRed, HcRed1), Anemonia cyan fluorescent proteins (AmCyan, AmCyan1), Zoanthus green fluorescent proteins (ZsGreen, ZsGreen1), Zoanthus yellow fluorescent proteins (ZsYellow, ZsYellow1), their fluorescence-enhancement variants, their peptide destabilization variants, and the like; Renilla reniformis green fluorescent protein (Vitality hrGFP), its fluorescence-enhancement variants, its peptide destabilization variants, and the like; and Great Star Coral fluorescent proteins, such, e.g., Montastrea cavernosa fluorescent protein (Monster Green^{®} Fluorescent Protein), its fluorescence-enhancement variants, its peptide destabilization variants, and the like. One skilled in the art understands that these and a variety of other fluorescent proteins can be useful as a fluorescent protein in aspects of the invention, see, e.g., [23]. One skilled in the art understands that these and many other fluorescent proteins, including species orthologs and paralogs of the above described naturally occurring fluorescent proteins as well as engineered fluorescent proteins can be useful as a fluorescent protein disclosed in aspects of the present specification. However, further examples of fluorescent labels and other labels that can be used in the context of immunoassays as well as the method of the present invention are known to the skilled person. Secondary detection molecules such as secondary antibodies or linked to fluorochromes can be obtained commercially, e.g., goat F(ab')2 anti-human IgG-FITC is available from Tago Immunologicals (Burlingame, Calif.).

Indirect labels include various enzymes well-known in the art, such as horseradish peroxidase (HRP), alkaline phosphatase (AP), β-galactosidase, urease, and the like. A horseradish-peroxidase detection system can be used, for example, with the chromogenic substrate tetramethylbenzidine (TMB), which yields a soluble product in the presence of hydrogen peroxide that is detectable at 450 nm. An alkaline phosphatase detection system can be used with the chromogenic substrate p-nitrophenyl phosphate, for example, which yields a soluble product readily detectable at 405 nm. Similarly, a β-galactosidase detection system can be used with the chromogenic substrate o-nitrophenyl-β-D-galactopyranoside (ONPG), which yields a soluble product detectable at 410 nm.

A signal from the direct or indirect label can be analyzed, for example, using a spectrophotometer to detect color from a chromogenic substrate; a radiation counter to detect radiation such as a gamma counter for detection of ¹²⁵I; or a fluorometer, photomultiplier or photodiode to detect fluorescence in the presence of light of a certain wavelength. For detection of enzyme-linked antibodies, a quantitative analysis of the amount of marker levels can be made using a spectrophotometer such as an EMAX Microplate Reader (Molecular Devices; Menlo Park, Calif.) in accordance with the manufacturer's instructions. If desired, the assays of the present invention can be automated or performed robotically, and the signal from multiple samples can be detected simultaneously.

As examples of means for detecting the label in the method of the present invention, a variety of immunoassay techniques, including competitive and non-competitive immunoassays, can be used to determine the presence or level of one or more markers in a sample (see, e.g., [24]). The term "immunoassay" encompasses techniques including, without limitation, enzyme immunoassays (EIA) such as enzyme multiplied immunoassay technique (EMIT), enzyme-linked immunosorbent assay (ELISA), antigen capture ELISA, sandwich ELISA, IgM antibody capture ELISA (MAC ELISA), and microparticle enzyme immunoassay (MEIA); capillary electrophoresis immunoassays (CEIA); radioimmunoassays (RIA); immunoradiometric assays (IRMA); fluorescence polarization immunoassays (FPIA); and chemiluminescence assays (CL). If desired, such immunoassays can be automated. Immunoassays can also be used in conjunction with laser induced fluorescence (see, e.g., [25], [26]). Liposome immunoassays, such as flow-injection liposome immunoassays and liposome immunosensors, are also suitable for use in the present invention (see, e.g., [27]).

Immunoassays come in many different formats and variations. Immunoassays may be run in multiple steps with reagents being added and washed away or separated at different points in the assay, and such multi-step assays are often called separation immunoassays or heterogeneous immunoassays. Due to the step of separating the liquid phase and the on the solid phase-coupled capture molecules after the incubation step, the method of the present invention corresponds to such a heterogeneous assay. Such heterogeneous assays can be further subdivided into competitive and non-competitive assays.

A competitive immunoassay is an assay, wherein - in contrast to a sandwich assay - competitor detection molecules, such as antigens or antibodies, which may be labeled, are added to the incubation of the capture molecules and the sample comprising the target molecules. This (labeled) detection molecule is in competition with the target molecule for binding to the capture molecule, for example to form the antigen-antibody complexes, on the solid phase. The amount of bound labeled detection molecules is dependent on the amount of target molecules present in the incubation mix. In other words, in a competitive assay the unlabeled analyte (target molecule) in a sample competes with labelled analyte (detection molecule) to bind an antibody or other capture molecule. After the incubation, the unbound analyte molecules (labeled and unlabeled) are separated from the solid phase and the capture molecules. In competitive heterogeneous assays of the state of the art, the labelled analytes which have bound to the capture molecules on the solid phase are measured, and this requires further washing steps of the solid phase. In contrast, the method of the present invention is detecting the unbound, labeled analyte detection molecule in the liquid phase after separation, while the liquid phase may still be in the same reaction well where the separation took place. Accordingly, it is possible to perform the assay of the invention without requiring any washing steps. By detecting the unbound competitive detection molecule (for example antigen or antibody) the amount or concentration of the target molecule in the sample can be determined after the bound-free phase is separated from the bound phase.

A sandwich assay differs from a competitive assay because a target molecule is detected through binding of a capture molecule as well as of a detection molecule to the target molecule. Preferably, the sandwich assay is an immunoassay, wherein two antibodies (or antigens) are used. Antibody 1 (antigen 1) may be immobilized on the solid phase as a capture molecule, which binds to an epitope of the target molecule (analyte). A second antibody 2 (antigen 2) may be labeled for detection (or carries a receptor or binding site for another labeled molecule/entity) and binds to a different epitope of the target molecule as compared to antibody 1 (antigen 1). The amount of bound detection molecules is dependent on the amount of sample antigens (antibodies). After the complex of the capture molecule, the target molecule and the detection molecule is formed (for example as a complex of two antibodies binding to different epitopes of an antigen) the bound-free phase (comprising the unbound second labeled antibody 2 (antigen 2)) is separated from the bound phase (comprising the complex of the capture molecule, the target molecule and the detection molecule). In sandwich heterogeneous assays of the state of the art, the labeled detection molecules which have bound to the target molecule and capture molecules on the solid phase are measured, and this requires further washing steps of the solid phase. In contrast, the method of the present invention is detecting the unbound labeled detection molecules in the liquid phase after separation, in embodiments while still being in the same reaction well where the separation took place, and yet without requiring any washing steps. By detecting the unbound labeled detection molecules (for example antigen or antibody) the amount or concentration of the target molecule in the sample can be detected after the bound-free phase is separated from the bound phase.

In the context of the present invention, clusters of beads consist of several smaller beads, that are assembled to form a larger bead by forming a cluster or agglomerate of smaller beads and may also be referred to as colloidal beads.

A washing step in the sense of the present invention relates to the washing of the solid phase after the separation of the solid phase-coupled capture molecules comprising the bound target molecules and/or detection molecules from the buffer solution. Such washing steps are required in known state-of-the-art heterogeneous immunoassays since these assays are based on detecting the presence of bound detection molecules on the solid surface after incubation. Without such washing steps using suitable wash buffers, that mostly differ from the buffer solutions employed during the co-incubation step for supporting the binding reaction, unspecific binding of detection molecules to the solid surface often leads to faulty results. Even in the case of membrane or immunochromatographic based assays, at least some buffer is needed for fluid propulsion along the lateral flow stripe. However, the method of the present invention has the advantage that such washing steps are not required since it is surprisingly possible to determine the presence and/or concentration of the target molecule by detecting the detection molecules remaining in the liquid phase after the co-incubation step and separation of the liquid phase from the solid phase, the procedure itself being surprisingly reproducible even though all the aforementioned steps can be performed in the same reaction well.

As used herein, a one-step (immuno)assay refers to an assay, in which the complex forming reagents, which comprise in the context of the present invention the capture molecules, the target molecules and the detection molecules, are added to the buffer solution in one step. This means, that the reagents are added about simultaneously or within a short period of time, preferably within seconds. Accordingly, incubation of the reagents taking part in the detection reaction occurs about simultaneously for all reagents involved, and no sequential incubation periods or steps are performed in a one-step assay. For example, if the solid phase comprising the coupled capture molecules are already present in the buffer solution, the target molecules and the detection molecules are added in one step at about the same time. The complex is formed while all reagents needed for detection are in the buffer solution.

An incubation, as used herein, refers to a period of time wherein certain reagents or molecules are present in the buffer solution at the same time to perform the binding reaction required for detecting the presence or absence of the target molecules. Accordingly, the incubation time is the duration of time during which the specific reaction between the capture molecule and the target molecule and/or the detection molecule takes place and the binding of the capture molecule to the target molecule and/or the detection molecule may take place. For example, during the incubation an antigen-antibody complex may be formed.

As used herein, the term "buffer solution" refers to a liquid, which is suitable for supporting the binding reaction between the capture molecules and the target molecules and/or the detection molecules. During incubation, the sample suspected to contain one or more target molecules, the buffer solution and potentially the liquids of the components of the incubation step form a liquid phase. The buffer solution can be selected and determined on the basis of the specific reaction to be supported and the conditions of the assay. The exact composition of the buffer solution may have to be adjusted specifically for the incubation and detection reaction to be carried out and may be influenced by factors such as the kind of sample to be analyzed, the binding characteristics of the specific capture molecule to the target molecule and the binding characteristics of the specific detection molecules to the target and/or the capture molecules. A person skilled in the art is able to select a suitable buffer solution to be used in the context of a specific method of the present invention.

In the context of the present invention, the separated buffer solution may also be referred to as the separated supernatant or the bound-free phase. After the complex of the capture molecule and the target molecule and/or the detection molecule is formed, for example in form of an antigen-antibody complex, and is bound to the solid phase due to the coupling of the capture molecules, the surplus molecules and any other material that did not bind to the capture molecules are left in the liquid phase. The surplus molecules suspended in the buffer that can be separated from the complex consisting of the solid phase and the molecules bound to the solid phase form the bound-free phase. The term "bound phase" refers to the solid phase and the complexes of the solid phase-coupled capture molecules with the target or detection molecules, such as for example antigen-antibody complexes that are formed on the surface of the solid phase.

A capture molecule that is coupled, linked or connected to a solid phase may be referred to as a solid phase-coupled capture molecule. A solid phase refers to a solid surface on which capture molecules, such as antibodies or antigens, can be immobilized. After incubation of the solid phase-coupled capture molecules, the target molecules and the detection molecules, the solid phase can be separated from the bound-free phase/liquid phase/supernatant.

Suitable solid phases for coupling capture molecules comprise, without limitation, solid beads or particles or a solid surface, such as the surface of a microtiter plate or a plate or any type of wall, used in the context of a microfluidic system. A microtiter plate may be a plate made out of plastic that has wells on its surface that can be used to run an immunoassay reaction. A solid particle or bead can be for example a magnetic bead, a microbead, a nanobead or a nanoparticle, such as a metal nanoparticle comprising for example gold, silver, gold, ceramic or glass.

The nature of the step of separating the solid phase from the buffer solution depends on the kind of solid phase used. For example, in case of a fixed solid surface, such as a microtiter plate or a surface of a microfluidic device, separation occurs practically automatically, since the capture molecules are fixed on the plate surface and the buffer solution forms a supernatant. Removal of the separated liquid phase (or a part thereof) from the capture-molecules can either occur manually, or automatically (e.g. with robotic pipette), or by flow through a microfluidic channel if the solid phase is a microfluidic surface. In case of beads serving as a solid phase in the sense of the method of the invention, separation may occur with the aid of magnetic, gravitational, centrifugal or electrophoretic forces or sedimentation for example. If magnetic beads serve as a solid phase for coupling the capture molecules, a permanent or electromagnetic field/a permanent or electromagnet can be used to separate the beads from the buffer, wherein the beads will be fixed at the location of a magnet, for example. After separation, removal of the liquid phase from the reaction well with the fixed beads, which may be fixed on the wall of a reaction chamber or well due to the magnetic force, is possible, for example. In case of micro- or nanobeads but with sufficiently higher density than the liquid phase (e.g. glass or ceramic, or the like) serving as solid phase for coupling the capture molecules, separation may occur through centrifugation, wherein the beads may be for example sedimenting to the bottom of the reaction chamber. Optionally, the liquid phase can be removed as the supernatant after separation by for example centrifugation. Electrophoretic separation may also be a possibility, for example in case of using charged particles as a solid phase. Due to different properties of beads it is also possible to use one kind of beads as a solid phase for coupling the capture molecules, and to use a different kind of bead as a label on a detection molecule.

### FIGURES

The invention is further described by the following figures. These are not intended to limit the scope of the invention, but represent preferred embodiments of aspects of the invention provided for greater illustration of the invention described herein.

### Brief description of the figures:

**Figure 1****:** Illustration of an immunoassay workflow according to the method of the present invention (as example a competitive immunoassay is shown).
**Figure 2****:** Process chain with immunoassay steps of an example of the method of the present invention.
**Figure 3****:** Intensity of remaining fluorescent beads in supernatant (in sandwich format).
**Figure 4****:** Intensity of remaining fluorescent beads in supernatant (standard curve in competitive format).
**Figure 5****:** Concentration CV range of bound-free phase assay.
**Figure 6****:** Measurement result of a certified reference material (CRM) here human serum with a certified value of 41.2 mg/l and an uncertainty range of ± 2.5 mg/l with the immunoassay method presented in this invention.

### Detailed description of the figures:

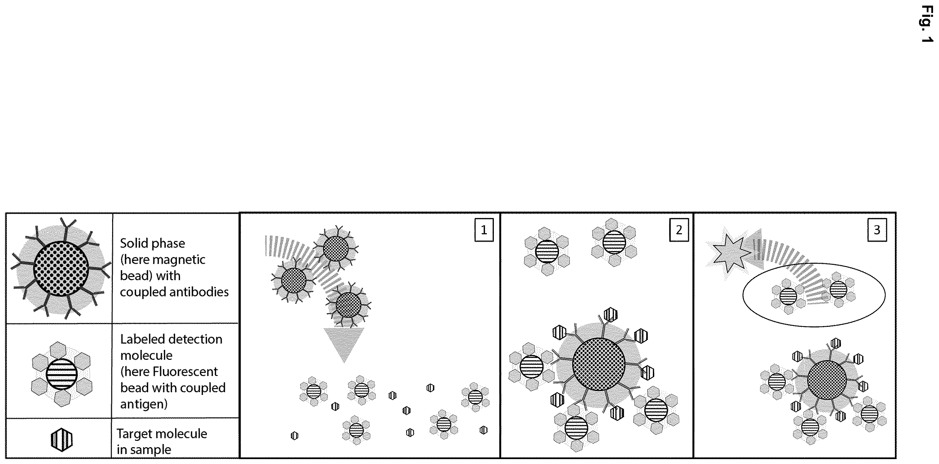

**Figure 1****:** (1) Magnetic beads (MB) are added to the fluorescent beads (FB) and the sample. (2) The antigens on the FB and in the sample react with the antibodies on the surface of the MB. (3) After the antigen-antibody complex is formed the phases are separated and bound-free phase (supernatant) with the surplus FB is detected. The number of surplus FB and, consequently, the detected signal are dependent on the amount of antigen in the sample due to the competition over the binding sites on the antibodies.

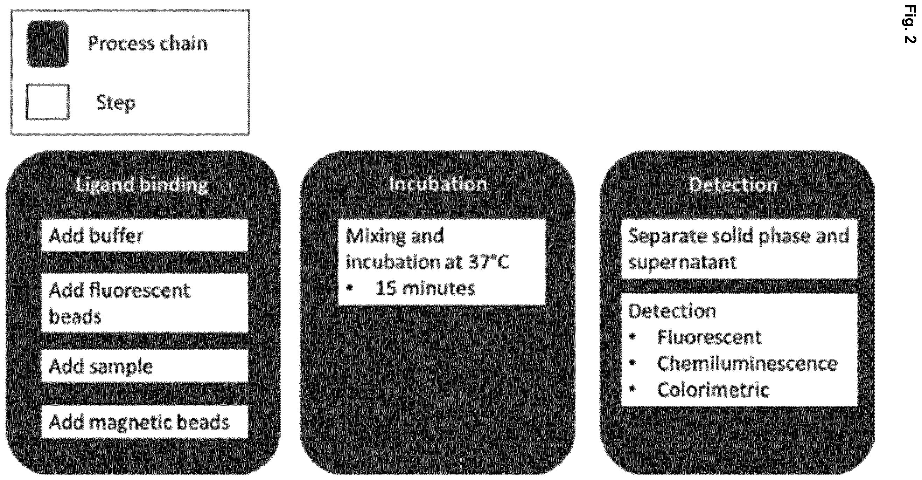

**Figure 2****:** This example of an immunoassay according to the present invention contains seven steps. The preparation of the fluorescent and magnetic beads are not included in this schematic as they are not part of the immunoassay workflow itself.

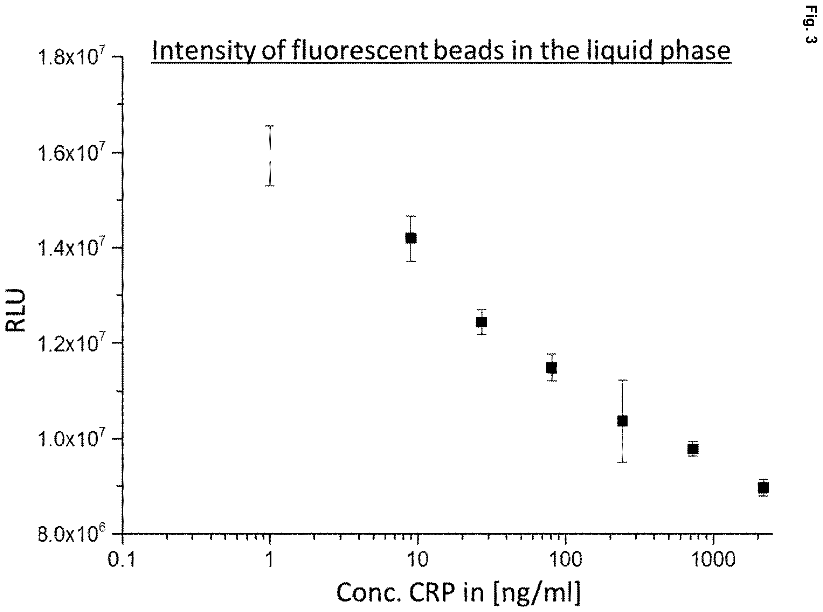

**Figure 3****:** Example of application of the method of this invention in sandwich CRP immunoassay (low concentration ranges). For this experiment fluorescent beads with a diameter of 0.5 µm (Merck former Sigma Aldrich, Germany) were used. The protocol principle for the sandwich assay (not described in this document) is the same as described for the competitive assay but two antibodies (R&D systems a biotechne brand, USA) are used to be able to form a sandwich. The capture antibody is immobilized on the magnetic beads (Thermo Fisher, USA). The detection antibody is immobilized on the fluorescent bead. This assay is a one-step sandwich assay.

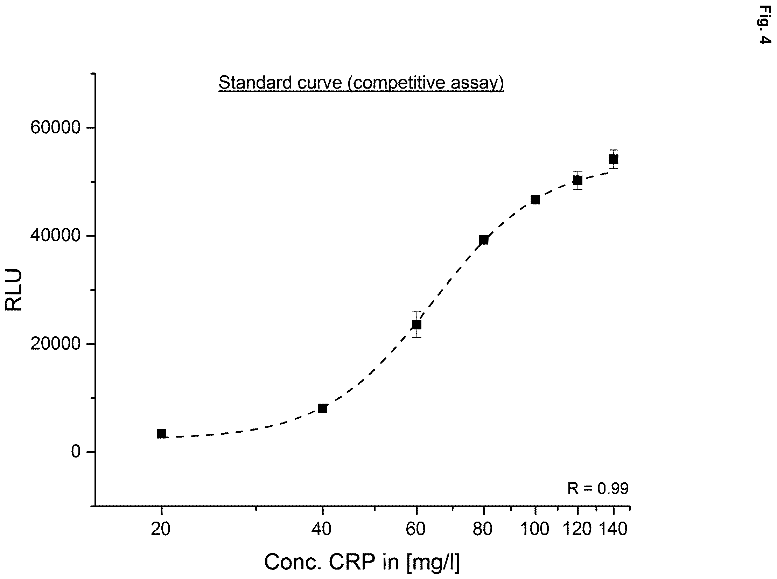

**Figure 4****:** Example of application of the method of this invention in competitive CRP immunoassay (high concentration ranges).This figure shows a standard curve of the CRP assay conducted with the protocol described in the section "EXAMPLES". The standard curve was created with a repetition of three assays for each value.

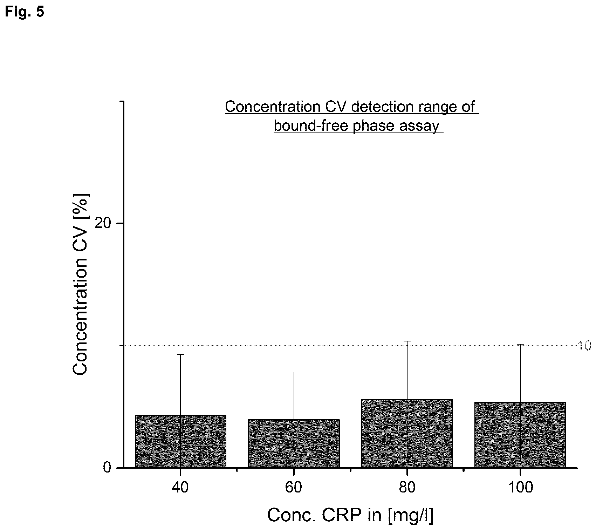

**Figure 5****:** An overview of the concentration CV in % over the CRP concentration range between 30 mg/l and 100 mg/l. The concentration values were calculated from the measured intensity by creating a standard curve with 5 repeated measurements for human serum samples with spiked CRP with the values 20 mg/l, 40 mg/l, 60 mg/l, 80 mg/l and 100 mg/l. The measurement data with their mean values and standard deviations were fitted with a sigmoidal function. This function was used to calculate the measured concentration of 16 repetitions for human serum samples with spiked CRP with the values 40 mg/l, 60 mg/l, 80 mg/l and 100 mg/l.

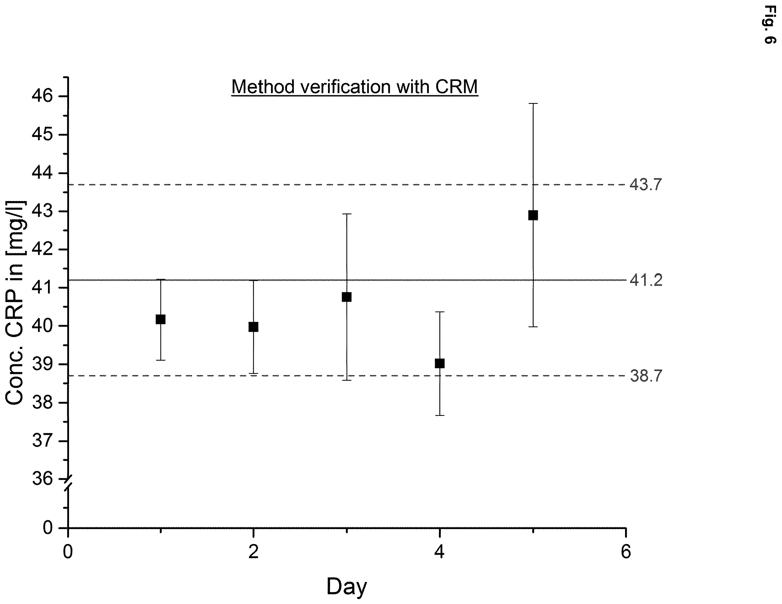

**Figure 6**: A certified reference material (CRM) (ERM-DA474/IFCC, Joint Research Centre (JRC) European Commission) here human serum with a certified value of 41.2 mg/l and an uncertainty range of ± 2.5 mg/l of CRP was tested with the CRP assay conducted with the protocol described in the section "EXAMPLES". The assay was conducted on 5 different days, each with 5 repetitions. To calculate the concentration from the measured intensity a standard curve created with the same material batch (magnetic beads, fluorescent beads, assay buffer) was used, which was created with the same method as described in the description of Figure 5, but here with 3 repeated measurements for each value. Figure 6 shows the mean value of the 5 repeated measurements of the certified reference material with their standard deviation for each day. The line at the value 41.2 mg/l represents the certified value of the sample and the dashed lines at 38.7 mg/l and 43.7 mg/l show the range covered by the certified material considering its uncertainty.

### EXAMPLES

The invention is further described by the following examples. These are not intended to limit the scope of the invention, but represent preferred embodiments of aspects of the invention provided for greater illustration of the invention described herein. The examples relate to a method for an immunoassay, which provides information about an antigen (or antibody) that is assumed to be present in a liquid sample, by detecting it in the bound-free phase (supernatant) of the immunoassay. With this approach it is possible to avoid the washing steps that are needed in other immunoassay approaches known in the art. Combined with a bead-based assay approach this immunoassay requires only a short incubation time and provides the possibility of measuring high as well as low concentration ranges. Furthermore, fewer handling steps compared to other immunoassay approaches are required. Accordingly, the method of the invention is ideal to be performed in an automated or semi-automated system, and/or an automated or semi-automated microfluidic system. The total number of steps for performing the method of the invention depends for example on the platform that is used. The preparation steps described in this example are for a competitive bead-based assay approach where a microtiter plate well is used only as a reaction chamber with no immobilized antibodies or other reagents on the microtiter place surface.

The example provided herein is a one-step, competitive and bead-based immunoassay for the detection of CRP in human serum. The concept is also applicable to different types of immunoassays for example one-step or two-step sandwich assays with different detection methods, such as, but not limited to, fluorescence, chemiluminescence, absorption or colorimetry.

In this example, the reaction volume consists of a sample containing the analyte antigen, a competitive antigen conjugated with a label entity, the assay buffer (all aforementioned forming the liquid phase), magnetic beads conjugated with antibodies (forming the solid phase). For this assay, the magnetic beads (MB) M280 Dynabeads Tosylactivated (Thermo Fisher Scientific, USA) were used as the solid phase, where the complex is formed. This assay uses polyclonal antibodies, but monoclonal antibodies were also tested and worked as well. The competitive antigen is coupled onto the fluorescent beads (FB) acting as the label entity. For this assay, FluoSpheres Carboxylate-Modified Microspheres with a diameter of 0.2 µm and excitation/emission wavelengths of 580 nm/605 nm (Thermo Fisher Scientific, USA) were used and coupled with native CRP antigen (United State Biological, USA). Fluorescent beads from another supplier with different diameters were tested and worked as well.

These coupled FluoSphere beads are added to the assay buffer together with the sample. In the next step the magnetic beads with the antibodies on their surface are added to the buffer-FB-sample mixture **(****Figure 1** **(1)).** If the to be detected antigen is present in the sample, it will compete with the competitive antigen that is coupled on the surface of the FB and an antigen-antibody complex with either the sample antigen or the FB-coupled detection antigen is formed **(****Figure 1** **(2))** during the incubation at 37 °C for 15 minutes. The amount of surplus FB-coupled detection antigen (bound-free phase) that has not bound to the MB-capture antibodies is dependent on the amount of antigen that is present in the sample. Thus, with separating the bound-free phase (in liquid phase) from the solid phase (complex that reacted onto the surface of the magnetic particle), the fluorescent signal of the surplus FB-coupled detection antigen can be detected and the concentration of the CRP in the sample can be determined **(****Figure 1** **(3)).** With that the assay also enables color multiplexing. There is no washing step needed, and the separation can be done by, but is not limited to, magnetic forces, centrifugal forces, electrophoretic forces, or biochemical adsorption.

**Figure 2** summarizes all steps required for the example of the method of the present invention. The framework for that can be a bead-based approach like the one presented above. The assay presented herein also allows non-bead-based approaches where the solid phase can be the bottom of a microtiter plate or other suitable surfaces. The signal to be detected is also not limited to derive from fluorescence beads. Fluorescent dyes, or quantum dots, or approaches like chemiluminescence, enzymatic reactions work as well, although some approaches would increase the number of steps (in the detection phase, not in the incubation phase where the method of the invention retains its advantages independently from the detection method) due to additional reagents that have to be added for the signal generation.

The method of the present invention was created for an easy automation, for example but not limited to, a centrifugal microfluidic platform. The assay as described in the present non-limiting example combines short incubation time, only seven steps in total, one liquid reagent that has to be stored (no washing steps and consequently no washing buffer(s) to be stored), small volumes and no additional material like membranes for the detection.

The method is described in an example of a CRP-immunoassay. There may be four main parameters that can influence the performance of the immunoassay after the reagents like antibodies, antigen and buffers are fixed and optimized: (i) the incubation time, (ii) the sample volume, (iii) the ratio of the amount of magnetic beads to the amount of fluorescent beads, and (iv) the incubation temperature. These parameters can be used to adapt the detection range, linear range and slope of the standard curve and, consequently, the sensitivity. With the incubation temperature it is also possible to adjust the duration of the incubation and also the slope of the linear range.

### Materials and methods of the examples

### Microtiter plate

A non-binding black microtiter plate (Greiner Bio-One GmbH, Austria) is blocked with a blocking buffer (PBS/BSA 5%) to prevent non-specific adsorption of the reagents on the microtiter plate surface. The steps are:
- Fill well completely with PBS/BSA 5%
- Block for 30 min at room temperature (RT) or over night at 8 °C
- 3 x wash with the wash buffer (PBS) by filling the wells completely, wait for 2 min and carefully shake the plate while waiting
- Remove washing buffer and dry the plate carefully on a laboratory paper towel by tapping the plate on it.

### Magnetic beads

The magnetic beads M280 tosylactivated Dynabeads (14203, Thermo Fisher Scientific, USA) are used as the solid phase. CRP antibodies (A80-125A, Bethyl Laboratories Inc. USA) are coupled on their surface. The protocol is given by the supplier (Thermo Fisher Scientific, USA) [28].

### Fluorescence beads

The fluorescence beads (F8812, Thermo Fisher Scientific, USA) are used as label of the detection molecule (the competitive antigen). The coupling protocol to immobilize highly purified native CRP antigen (C7907-26, United State Biological, USA) onto the surface of the FluoSpheres is provided by the supplier [29].

### Results of the examples

For the proof-of-concept, one-step bead-based assays are presented. These assays are able to cover small detection ranges (3 ng/ml to 2000 ng/ml, in sandwich format, **Figure 3**) as well as high detection ranges (28.6 mg/l to 140 mg/l in competitive format, **Figure 4**) and have fast incubation duration of 15 min or less. Importantly, the exclusion of any washing does not have any negative influence on the reproducibility, as we could show an inter assay coefficient of variation (CV) with 4.3 % and an inter assay CV with 4.1 % for the example of the competitive CRP assay measured with a certified reference material (ERM-DA474/IFCC, European commission, Reference Materials Unit). It is important to optimize the volumes of the reagents used in the assay protocol. The protocol that is described below is the optimized protocol for a CRP immunoassay with human serum as sample material and a measuring range of 28.6 mg/l to 140 mg/l in a non-binding black microtiter plate (Greiner Bio-One GmbH, Austria).

### Protocol for a competitive assay with antigen (detection molecule) coupled on the FluoSpheres (label) and antibody (capture molecule) coupled on the Dynabeads (solid phase)

### Immunoassay (total volume 75 µl)

- Bring the reagents to room temperature
- Add 50 µl assay buffer
- Add 10 µl FluoSphere beads (2 mg/ml)
- Add 5 µl sample (native human CRP-free serum)
- Add 11.5 µl Dynabeads (20 mg/ml stock concentration)
- Incubate for 15 min at 37 °C on a shaker
- Separate the magnetic beads with a magnet from the bound-free phase (supernatant)
- Take 50 µl of the supernatant
- Measure the fluorescence

This assay shows an inter-assay CV of 4.1 % (considered as very good results in the microtiter plate) and an intra-assay CV of 4.35 % measured over 5 different days in a non-automated system with the CRM. **Figure 4** shows a standard curve for the CRP. Each measured concentration was repeated three times. The sample material was native human CRP-free serum (HyTest Ltd, Finland) spiked with a known concentration of native human CRP. The read out was done on a microtiter plate reader (Tecan Spark 10M, Tecan Trading GmbH, Switzerland). **Figure 5** shows the calculated concentration CV for 40 mg/l, 60 mg/l, 80 mg/l and 100 mg/l. All mean CV values are lower than 10 % with the highest value for 80 mg/l being 5.6 % and the lowest value with 3.9 % for the 60 mg/l. These values were calculated as described in "Detailed description of the figures". This proves that the assay can be considered quantitive between 40 mg/l and 100 mg/l and therefore the absence of washing step does not influence the quality of the assay. **Figure 6** shows the measurement results of the CRM (ERM-DA474/IFCC, Joint Research Centre (JRC) European Comission) with a certified value of 41.2 mg/l and an uncertainty range of ± 2.5 mg/l with the method described in "Detailed description of the figures". The line at the value 41.2 mg/l represents the certified value of the sample and the dashed lines at 38.7 mg/l and 43.7 mg/l show the range covered by the certified material considering its uncertainty. All mean values measured on each day are within the range of the CRM which proves the functionality of the method. It also proves that the method is not in any way influenced by different samples. In conclusion, the assay concept was evaluated and is proven to work. The CRP competitive assay is quantitative with CVs clearly under 10 % and was shown to be used with undiluted sample material.

### References

[1] Welcome trust, HM Government, TACKLING DRUG-RESISTANT INFECTIONS GLOBALLY: FINAL REPORT AND RECOMMENDATIONS: THE REVIEW ON ANTIMICROBIAL RESISTANCE. Available: https://amr-review.org/sites/default/files/160518_Final%20paper_with%20cover.pdf (2018, Apr. 25).
[2] Cals, JWL. et al, "Effect of point of care testing for C reactive protein and training in communication skills on antibiotic use in lower respiratory tract infections: cluster randomised trial", BMJ (Clinical research ed.), vol. 338, pp. b1374, 2009.
[3] Aabenhus, R. et al, "Biomarkers as point-of-care tests to guide prescription of antibiotics in patients with acute respiratory infections in primary care", The Cochrane database of systematic reviews, no. 11, pp. CD010130, 2014.
[4] Nargis, W. et al, "Procalcitonin versus C-reactive protein: Usefulness as biomarker of sepsis in ICU patient", International journal of critical illness and injury science, vol. 4, no. 3, pp. 195-199, 2014.
[5] Dittrich, S. et al, "Target Product Profile for a Diagnostic Assay to Differentiate between Bacterial and Non-Bacterial Infections and Reduce Antimicrobial Overuse in Resource-Limited Settings: An Expert Consensus", PloS one, vol. 11, no. 8, pp. e0161721, 2016.
[6] Kazarian, A. et al, "Testing breast cancer serum biomarkers for early detection and prognosis in pre-diagnosis samples", British journal of cancer, vol. 116, no. 4, pp. 501-508, 2017.
[7] Andriole, G. et al, "The Case for Prostate Cancer Screening with Prostate-Specific Antigen", European Urology Supplements, vol. 5, no. 12, pp. 737-745, 2006.
[8] Baskić, D. et al, "Clinical evaluation of the simultaneous determination of CA 15-3, CA 125 and sHER2 in breast cancer", Biomarkers: biochemical indicators of exposure, response, and susceptibility to chemicals, vol. 12, no. 6, pp. 657-667, 2007.
[9] K. Mitsakakis and E. Gizeli, "Detection of multiple cardiac markers with an integrated acoustic platform for cardiovascular risk assessment", Analytica chimica acta, vol. 699, no. 1, pp. 1-5, 2011.
[10] N. Bostanci and K. Bao, "Contribution of proteomics to our understanding of periodontal inflammation", Proteomics, vol. 17, no. 3-4, 2017.
[11] AH. Wu, "A selected history and future of immunoassay development and applications in clinical chemistry", Clinica chimica acta; international journal of clinical chemistry, vol. 369, no. 2, pp. 119-124, 2006.
[12] Vashist, S. et al, "One-step kinetics-based immunoassay for the highly sensitive detection of C-reactive protein in less than 30 min", Analytical biochemistry, vol. 456, pp. 32-37, 2014.
[13] Zhao, Y. et al, "C-reactive protein and interleukin 6 microfluidic immunoassays with on-chip pre-stored reagents and centrifugo-pneumatic liquid control", Lab on a chip, vol. 17, no. 9, pp. 1666-1677, 2017.
[14] Nobuo Hiratsuka, Fuji Photo Film Co,Ltd, "Competitive binding immunoassay process" Patent Number: US4868131A, 1989.
[15] Gordon C. Forrest, Philip R. P. Salacinski, Kenneth Siddle, Serono Diagnostics Limited, "IMMUNOASSAY OF ANTIGENS" Patent Number: US4659678A, 1987.
[16] Henry J. Jeong, Judith I. Blakemore, Nathan Lewin, BIO-RAD Laboratories, Inc, "Single-incubation two-site immunoassay" Patent Number: US4244940A, 1981.
[17] William Jeffrey Allard, Kwok K. Yeung, Zeqi Zhou, Bayer Corporation, "Determination of CPSA" Patent Number: US5840501, 1998.
[18] Hon-Peng P. Lau, Dade Behring Inc, "Use of chromatographic supports having immobilized flocculating agent in separating microparticles" Patent Number: US5447870, 1995.
[19] Strohmeier, O. et al, "Centrifugal microfluidic platforms: advanced unit operations and applications", Chemical Society reviews, vol. 44, no. 17, pp. 6187-6229, 2015.
[20] Douglas Robert Evans, Gerald John Allen, Carolyn Jennifer Ruddell, PLATFORM DIAGNOSTIC LIMITED, "Saturation assay" Patent Number: WO 2008/056165 A1, 2008.
[21] Fernandez-Baldo MA. et al, "Determination of Ochratoxin A in apples contaminated with Aspergillus ochraceus by using a microfluidic competitive immunosensor with magnetic nanoparticles", Analyst, no. 13, pp. 2756-2762, 2011.
[22] Tang, M. et al, "A Review of Biomedical Centrifugal Microfluidic Platforms", Micromachines, vol. 7, no. 2, p. 26, 2016.
[23] J. Lippincott-Schwartz and G. Patterson, "Development and use of fluorescent protein markers in living cells", Science (New York, N.Y.), vol. 300, no. 5616, pp. 87-91, 2003.
[24] C. H. Self and D. Cook, "Advances in immunoassay technology", Current Opinion in Biotechnology, vol. 1996, no. 7, pp. 60-65, 1996.
[25] D. Schmalzing and W. Nashabeh, "Capillary electrophoresis based immunoassays: a critical review", Electrophoresis, vol. 18, no. 12-13, pp. 2184-2193, 1997.
[26] Bao, JJ. et al, "Capillary electrophoretic immunoassays", Journal of Chromatography B: Biomedical Sciences and Applications, vol. 1997, no. 1-2, pp. 463-480, 1997.
[27] Rongen, HA. et al, "Liposomes and immunoassays", Journal of Immunological Methods, vol. 1997, no. 2, pp. 105-133, 1997.
[28] Invitrogen by Life Technologies, Dynabeads® M-280 Tosylactivated. Available: https://assets.thermofisher.com/TFS-Assets/LSG/manuals/dynabeads_m280tosylactivated_man.pdf (2018, Apr. 25).
[29] I. Molecular Probes, Working With FluoSpheresAE Fluorescent Microspheres. Available: https://assets.thermofisher.com/TFS-Assets/LSG/manuals/mp05001.pdf (2018, Apr. 25).

## Claims

1. A method for detecting a target molecule in a sample, **comprising** the sequential steps of
a. incubating at the same time in a buffer solution in a reaction well
i. the sample suspected to contain one or more target molecules,
ii. capture molecules suitable for binding to the target molecules, wherein the capture molecules are coupled to beads (capture-beads),
iii. a defined amount of detection molecules suitable for binding to the target molecules and/or the capture molecules, wherein the detection molecules are coupled to beads (detection-beads),
wherein the capture-beads are separable from the detection-beads,
b. separating in the reaction well the capture-beads from the buffer solution comprising unbound detection-beads, and
c. detecting remaining unbound detection-beads in the buffer solution, which has been separated from the capture-beads, wherein the detection of remaining unbound detection-beads occurs in the same reaction well as the incubation and separation by means of a label that does not require additional reagents for signal generation,
wherein the method does not comprise a washing step.

2. Method according to any of the preceding claims, wherein the label that does not require additional reagents for signal generation is a fluorescent tag.

3. Method according to any of the preceding claims, wherein the method does not require more than one buffer solution.

4. Method according to any of the preceding claims, wherein the method is performed in an automated system, preferably a microfluidic system, more preferably a centrifugal microfluidic system.

5. Method according to any of the preceding claims, wherein the capture-beads and/or the detection-beads are single compact beads.

6. Method according to any of the preceding claims, wherein the capture-beads have a higher density than the detection-beads and/or a diameter of at least 0.5 µm.

7. Method according to the preceding claim, wherein separating the capture-beads from the buffer solution occurs through magnetic, gravitational, centrifugal or electrophoretic forces, preferably through sedimentation.

8. Method according to any of the preceding claims, wherein
a. the capture molecule recognizes a first epitope comprised by the target molecule, and
b. the detection molecule recognizes a second epitope comprised by the target molecule (sandwich assay) or
c. the detection molecule comprises the first epitope recognized by the capture molecule (competitive assay).

9. Method according to any of the preceding claims, wherein the capture molecule comprises an antibody or fragments thereof or an antigen, and/or the detection molecule comprises an antibody or fragments thereof or an antigen.

10. Method according to any of the preceding claims, wherein the detection molecule comprises a label.

11. Method according to any of the preceding claims, wherein the incubation lasts for no more than 20 minutes and/or separation occurs in no more than 1 minute.

12. Method according to any of the preceding claims, wherein the assay provides quantitative results for detection of a target molecule.

13. Method according to any of the preceding claims, wherein two or more different target molecules comprised in the sample are detected in parallel, wherein
a. one or more target molecules are detected by a sandwich assay and one or more other target molecules are detected by a competitive assay, or
b. all target molecules are detected by a sandwich assay, or
c. all target molecules are detected by a competitive assay, or and
d. one or more of the target molecules are present in a high concentration and one or more other target molecules are present in a low concentration, or
e. all target molecules are present in a high concentration, or
f. all target molecules are present in a low concentration.

14. Use of a kit for detecting a target molecule in a sample according to the method of any of the preceding claims, wherein the kit comprises
a. capture-beads suitable for binding to the target molecules,
b. detection-beads suitable for binding to the target molecules and/or the capture-beads, wherein capture-beads and the detection-beads are single compact beads, the capture-beads are separable from the detection-beads, the capture-beads have a higher density than or equal density to the detection-beads and the capture-beads have a diameter of at least 0.5 µm,
c. a microfluidic system, preferably a centrifugal microfluidic system, configured for performing in one reaction chamber the method steps of
i. incubating at the same time in a buffer solution the sample suspected to contain one or more target molecules, the capture-beads and a defined amount of detection beads,
ii. separating in the reaction well the capture-beads from the buffer solution comprising unbound detection-beads, and
iii. detecting remaining unbound detection-beads in the buffer solution by means of a label that does not require additional reagents for signal generation,
d. and optionally a buffer solution and/or means for detecting the detection-beads in a buffer solution after
i. incubating in the buffer solution the capture-beads, the sample suspected to contain one or more target molecules and a defined amount of the detection-beads, and
ii. separating in the reaction well the capture-beads from the buffer solution comprising unbound detection-beads.

## Patentansprüche

1. Verfahren zum Nachweisen eines Zielmoleküls in einer Probe, die folgenden aufeinanderfolgenden Schritte **umfassend:**
a. gleichzeitiges Inkubieren in einer Pufferlösung in einem Reaktionsbehälter von Folgendem:
i. der Probe, von der vermutet wird, dass sie ein oder mehrere Zielmoleküle enthält,
ii. Fängermolekülen, die zur Bindung an die Zielmoleküle geeignet sind, wobei die Fängermoleküle an Kügelchen (Fängerkügelchen) gekoppelt sind,
iii. einer definierten Menge an Nachweismolekülen, die zur Bindung an die Zielmoleküle und/oder die Fängermoleküle geeignet sind, wobei die Nachweismoleküle an Kügelchen (Nachweiskügelchen) gekoppelt sind,
wobei die Fängerkügelchen von den Nachweiskügelchen trennbar sind,
b. Trennen der Fängerkügelchen in dem Reaktionsbehälter von der Pufferlösung, die ungebundene Nachweiskügelchen umfasst, und
c. Nachweisen von übrigen ungebundenen Nachweiskügelchen in der Pufferlösung, die von den Fängerkügelchen getrennt wurden, wobei der Nachweis von übrigen ungebundenen Nachweiskügelchen in demselben Reaktionsbehälter wie die Inkubation und Trennung mittels einer Kennzeichnung erfolgt, die keine zusätzlichen Reagenzien zur Signalerzeugung erfordert,
wobei das Verfahren keinen Waschschritt umfasst.

2. Verfahren nach einem der vorhergehenden Ansprüche, wobei die Kennzeichnung, die keine zusätzlichen Reagenzien zur Signalerzeugung erfordert, eine fluoreszierende Markierung ist.

3. Verfahren nach einem der vorhergehenden Ansprüche, wobei das Verfahren nicht mehr als eine Pufferlösung erfordert.

4. Verfahren nach einem der vorhergehenden Ansprüche, wobei das Verfahren in einem automatisierten System durchgeführt wird, vorzugsweise einem mikrofluidischen System, weiter bevorzugt einem zentrifugalen mikrofluidischen System.

5. Verfahren nach einem der vorhergehenden Ansprüche, wobei die Fängerkügelchen und/oder die Nachweiskügelchen einzelne kompakte Kügelchen sind.

6. Verfahren nach einem der vorhergehenden Ansprüche, wobei die Fängerkügelchen eine höhere Dichte als die Nachweiskügelchen und/oder einen Durchmesser von mindestens 0,5 µm aufweisen.

7. Verfahren nach dem vorhergehenden Anspruch, wobei das Trennen der Fängerkügelchen von der Pufferlösung durch magnetische, gravitative, zentrifugale oder elektrophoretische Kräfte, vorzugsweise durch Sedimentation, erfolgt.

8. Verfahren nach einem der vorhergehenden Ansprüche, wobei Folgendes gilt:
a. das Fängermolekül erkennt ein erstes Epitop, das von dem Zielmolekül umfasst wird, und
b. das Nachweismolekül erkennt ein zweites Epitop, das von dem Zielmolekül umfasst wird (Sandwich-Assay), oder
c. das Nachweismolekül umfasst das erste Epitop, das von dem Fängermolekül erkannt wird (kompetitiver Assay).

9. Verfahren nach einem der vorhergehenden Ansprüche, wobei das Fängermolekül einen Antikörper oder Fragmente davon oder ein Antigen umfasst und/oder das Nachweismolekül einen Antikörper oder Fragmente davon oder ein Antigen umfasst.

10. Verfahren nach einem der vorhergehenden Ansprüche, wobei das Nachweismolekül eine Kennzeichnung umfasst.

11. Verfahren nach einem der vorhergehenden Ansprüche, wobei die Inkubation nicht länger als 20 Minuten dauert und/oder die Trennung in nicht mehr als einer Minute erfolgt.

12. Verfahren nach einem der vorhergehenden Ansprüche, wobei der Assay quantitative Ergebnisse zum Nachweis eines Zielmoleküls bereitstellt.

13. Verfahren nach einem der vorhergehenden Ansprüche, wobei zwei oder mehr unterschiedliche Zielmoleküle, die in der Probe umfasst sind, parallel nachgewiesen werden, wobei
a. ein oder mehrere Zielmoleküle durch einen Sandwich-Assay nachgewiesen werden und ein oder mehrere andere Zielmoleküle durch einen kompetitiven Assay nachgewiesen werden oder
b. alle Zielmoleküle durch einen Sandwich-Assay nachgewiesen werden oder
c. alle Zielmoleküle durch einen kompetitiven Assay nachgewiesen werden oder
und
d. eines oder mehrere der Zielmoleküle in einer hohen Konzentration vorhanden sind und ein oder mehrere andere Zielmoleküle in einer niedrigen Konzentration vorhanden sind oder
e. alle Zielmoleküle in einer hohen Konzentration vorhanden sind oder
f. alle Zielmoleküle in einer niedrigen Konzentration vorhanden sind.

14. Verwendung eines Kits zum Nachweisen eines Zielmoleküls in einer Probe gemäß dem Verfahren nach einem der vorhergehenden Ansprüche, wobei das Kit Folgendes umfasst:
a. Fängerkügelchen, die zur Bindung an die Zielmoleküle geeignet sind,
b. Nachweiskügelchen, die zur Bindung an die Zielmoleküle und/oder die Fängerkügelchen geeignet sind,
wobei die Fängerkügelchen und die Nachweiskügelchen einzelne kompakte Kügelchen sind, die Fängerkügelchen von den Nachweiskügelchen trennbar sind, die Fängerkügelchen eine höhere Dichte als oder die gleiche Dichte wie die Nachweiskügelchen aufweisen und die Fängerkügelchen einen Durchmesser von mindestens 0,5 µm aufweisen,
c. ein mikrofluidisches System, vorzugsweise ein zentrifugales mikrofluidisches System, das zum Durchführen der folgenden Verfahrensschritte in einer Reaktionskammer konfiguriert ist:
i. gleichzeitiges Inkubieren der Probe, von der vermutet wird, dass sie ein oder mehrere Zielmoleküle enthält, der Fängerkügelchen und einer definierten Menge an Nachweiskügelchen in einer Pufferlösung,
ii. Trennen der Fängerkügelchen in dem Reaktionsbehälter von der Pufferlösung, die ungebundene Nachweiskügelchen umfasst, und
iii. Nachweisen übriger ungebundener Nachweiskügelchen in der Pufferlösung mittels einer Kennzeichnung, die keine zusätzlichen Reagenzien zur Signalerzeugung erfordert,
d. und optional eine Pufferlösung und/oder Mittel zum Nachweisen der Nachweiskügelchen in einer Pufferlösung nach
i. Inkubieren der Fängerkügelchen, der Probe, von der vermutet wird, dass sie ein oder mehrere Zielmoleküle enthält, und einer definierten Menge an Nachweiskügelchen in der Pufferlösung und
ii. Trennen der Fängerkügelchen in dem Reaktionsbehälter von der Pufferlösung, die ungebundene Nachweiskügelchen umfasst.

## Revendications

1. Procédé de détection d'une molécule cible dans un échantillon, **comprenant** les étapes séquentielles
a. d'incubation simultanée dans une solution tampon dans un puits de réaction
i. de l'échantillon suspecté de contenir une ou plusieurs molécules cibles,
ii. des molécules de capture aptes à se lier aux molécules cibles, dans lequel les molécules de capture sont couplées à des billes (billes de capture),
iii. d'une quantité définie de molécules de détection aptes à se lier aux molécules cibles et/ou aux molécules de capture, dans lequel les molécules de détection sont couplées à des billes (billes de détection),
dans lequel les billes de capture sont séparables des billes de détection,
b. de séparation dans le puits de réaction des billes de capture de la solution tampon comprenant les billes de détection non liées, et
c. de détection des billes de détection non liées restantes dans la solution tampon, qui a été séparée des billes de capture, dans lequel la détection des billes de détection non liées restantes se produit dans le même puits de réaction que l'incubation et la séparation au moyen d'un marqueur qui ne nécessite pas de réactifs supplémentaires pour la génération de signaux,
dans lequel le procédé ne comprend pas d'étape de lavage.

2. Procédé selon l'une quelconque des revendications précédentes, dans lequel le marqueur qui ne nécessite pas de réactifs supplémentaires pour la génération de signal est un marqueur fluorescent.

3. Procédé selon l'une quelconque des revendications précédentes, dans lequel le procédé ne nécessite pas plus d'une solution tampon.

4. Procédé selon l'une quelconque des revendications précédentes, dans lequel le procédé est exécuté dans un système automatisé, de préférence un système microfluidique, plus préférablement un système microfluidique centrifuge.

5. Procédé selon l'une quelconque des revendications précédentes, dans lequel les billes de capture et/ou les billes de détection sont des billes compactes uniques.

6. Procédé selon l'une quelconque des revendications précédentes, dans lequel les billes de capture ont une densité plus élevée que les billes de détection et/ou un diamètre d'au moins 0,5 µm.

7. Procédé selon la revendication précédente, dans lequel la séparation des billes de capture de la solution tampon se fait par des forces magnétiques, gravitationnelles, centrifuges ou électrophorétiques, de préférence par sédimentation.

8. Procédé selon l'une quelconque des revendications précédentes, dans lequel
a. la molécule de capture reconnaît un premier épitope constitué par la molécule cible, et
b. la molécule de détection reconnaît un second épitope constitué par la molécule cible (essai en sandwich) ou
c. la molécule de détection comprend le premier épitope reconnu par la molécule de capture (essai compétitif).

9. Procédé selon l'une quelconque des revendications précédentes, dans lequel la molécule de capture comprend un anticorps ou des fragments de celui-ci ou un antigène, et/ou la molécule de détection comprend un anticorps ou des fragments de celui-ci ou un antigène.

10. Procédé selon l'une quelconque des revendications précédentes, dans lequel la molécule de détection comprend un marqueur.

11. Procédé selon l'une quelconque des revendications précédentes, dans lequel l'incubation ne dure pas plus de 20 minutes et/ou la séparation se produit en moins d'une minute.

12. Procédé selon l'une quelconque des revendications précédentes, dans lequel l'essai fournit des résultats quantitatifs pour la détection d'une molécule cible.

13. Procédé selon l'une quelconque des revendications précédentes, dans lequel deux molécules cibles ou plus différentes comprises dans l'échantillon sont détectées en parallèle, dans lequel
a. une ou plusieurs molécules cibles sont détectées par un essai en sandwich et une ou plusieurs autres molécules cibles sont détectées par un essai compétitif, ou
b. toutes les molécules cibles sont détectées par un essai en sandwich, ou
c. toutes les molécules cibles sont détectées par un essai compétitif, ou
et
d. une ou plusieurs des molécules cibles sont présentes à une concentration élevée et une ou plusieurs autres molécules cibles sont présentes à une faible concentration, ou
e. toutes les molécules cibles sont présentes à une concentration élevée, ou
f. toutes les molécules cibles sont présentes à une faible concentration.

14. Utilisation d'un kit pour détecter une molécule cible dans un échantillon selon le procédé selon l'une quelconque des revendications précédentes, dans laquelle le kit comprend
a. des billes de capture aptes à se lier aux molécules cibles,
b. des billes de détection aptes à se lier aux molécules cibles et/ou des billes de capture,
dans lequel les billes de capture et les billes de détection sont des billes compactes uniques, les billes de capture sont séparables des billes de détection, les billes de capture ont une densité supérieure ou égale aux billes de détection et les billes de capture ont un diamètre d'au moins 0,5 µm,
c. un système microfluidique, de préférence un système microfluidique centrifuge, configuré pour effectuer dans une chambre de réaction les étapes de procédé
i. d'incubation simultanée dans une solution tampon de l'échantillon suspecté de contenir une ou plusieurs molécules cibles, des billes de capture et d'une quantité définie de billes de détection,
ii. de séparation dans le puits de réaction des billes de capture de la solution tampon comprenant les billes de détection non liées, et
iii. de détection des billes de détection non liées restantes dans la solution tampon au moyen d'un marqueur qui ne nécessite pas de réactifs supplémentaires pour la génération de signal,
d. et éventuellement une solution tampon et/ou des moyens pour détecter les billes de détection dans une solution tampon après
i. incubation simultanée dans une solution tampon des billes de capture, de l'échantillon suspecté de contenir une ou plusieurs molécules cibles et d'une quantité définie de billes de détection, et
ii. séparation dans le puits de réaction des billes de capture de la solution tampon comprenant les billes de détection non liées.
